# EUROPEAN PATENT SPECIFICATION

(11) **EP 3 578 266 B1**
(45) Date of publication and mention of the grant of the patent: **19.06.2024**
(21) Application number: 19173089.4
(22) Date of filing: 17.03.2014
(51) Int. Cl.: A61P 5/28, A61P 5/44, A61P 43/00, A61K 9/14, A61K 9/20, A61K 31/58, C07J 43/00, A61K 9/16, A61P 13/08, A61P 35/00, A61K 31/573

(54) **ABIRATERONE ACETATE FORMULATION**
ABIRATERON-ACETAT-FORMULIERUNG
FORMULATION D'ACÉTATE D'ABIRATÉRONE

(30) Priority: 15.03.2013 US 201361789141 P; 27.09.2013 US 201361883941 P
(43) Date of publication of application: 11.12.2019
(62) Divisional of application: 14765788.6
(73) Proprietor: Sun Pharmaceutical Industries Limited, Mumbai Maharashtra 400 063 (IN)
(72) Inventor: Bosch, William, H., Philadelphia, PA Pennsylvania 19112 (US); Norret, Marck, Hovea, 6071, West Australia (WA) (AU); Nemeth, Paul, Philadelphia, PA Pennsylvania 19112 (US); Callahan, Matt, Haverford, PA Pennsylvania 19041 (US)
(74) Representative: Harrison IP Limited

(56) References cited:
- WO-A1-2013/164473
- WO-A1-2014/009436
- CN-A- 102 743 393
- Telephone Anonymous: "7 Westferry Circus ● Canary Wharf ● London E14 4HB ● United Kingdom Committee for Medicinal Products for Human Use (CHMP) Assessment Report as adopted by the CHMP with all information of a commercially confidential nature deleted TABLE OF CONTENTS 1. Background information on th", JANSSEN-CILAG INTERNATIONAL N.V., 1 January 2011 (2011-01-01), page 8, XP055286152, Retrieved from the Internet: URL:http://www.ema.europa.eu/docs/en_GB/do cument_library/EPAR_-_Public_assessment_re port/human/002321/WC500112860.pdf [retrieved on 2016-07-06]

## Description

### Field of the Disclosure

The present disclosure relates to methods for producing particles of abiraterone acetate using dry milling processes as well as compositions comprising abiraterone acetate, medicaments produced using abiraterone acetate and to methods of treatment using a therapeutically effective amount of abiraterone acetate administered by way of said medicaments.

### Background

Poor oral bioavailability is a significant problem encountered in the development of therapeutic compositions, particularly those compositions containing a drug which is poorly soluble in water at physiological pH. A drug's oral bioavailability is the degree to which the drug is absorbed into the bloodstream after oral administration. Many factors affect oral bioavailability, including the form of dosage and the solubility and dissolution rate of the drug.

In therapeutic applications, poorly water-soluble drugs tend to be eliminated from the gastrointestinal tract before being completely absorbed into the circulation. They also tend to be absorbed slowly, which can result in slow onsest of therapeutic effect. In addition, poorly water-soluble drugs tend to be disfavored or even unsafe for intravenous administration due to the risk of particles of drug blocking blood flow through capillaries.

It is known that increasing the rate of dissolution of poorly soluble drugs will, in many cases, increase the rate and extent of their oral absorption. It is also known that the rate of dissolution of a particulate drug will increase with increasing surface area. One way of increasing surface area is decreasing particle size. Consequently, methods of making finely divided or sized drugs have been studied with a view to increasing the surface area and dissolution rates of drug particles used in pharmaceutical compositions.

Abiraterone ((3β)-17-(pyridin-3-yl) androsta-5, 16-dien-3-ol; CAS #: 154229-19-3; Formula: C₂₄H₃₁NO; Mol. Weight: 349.5 g/mol) is an inhibitor of CYP17 and thus interferes with the synthesis of androgens in the testes, adrenal glands and prostate tumor tissue. Abiraterone acetate (17-(3-Pyridyl)androsta-5, acetate; CAS #154229-18-2), a prodrug of abiraterone, is approved in the United States for treatment of castration-resistant prostate cancer. Abiraterone acetate is considered poorly water soluble.

Zytiga^{®} Tablets (250 mg) are approved in the United States in combination with prednisone for the treatment of patients with metastatic castration-resistant prostate cancer. The prescribing information for Zytiga^{®} tablets recommends 1,000 mg (4 x250 mg tablets) administered orally once daily in combination with prednisone (5 mg) administered orally twice daily. The European approval is for administration in combination with either prednisone or prednisolone. The prescribing information states that Zytiga^{®} must be taken on an empty stomach and that no food should be consumed for at least two hours before the dose is taken and for and for at least one hour after the dose is taken. The prescribing information explains that at a dose of 1,000 mg daily in patients with metastatic, castration resistant prostate cancer the steady-state values (mean ± SD) of Cmax were 226 ± 178 ng/mL and of AUC were 1173 ± 690 ng.hr/mL. A single dose (1000 mg) cross-over study of Zytiga in healthy subjects found that systemic exposure of abiraterone is increased when Zytgia^{®} is administered with food. Specifically, abiraterone Cₘₐₓ and AUC _{0-∞} were approximately 7- and 5-fold higher, respectively, when Zytiga^{®} was administered with a low-fat meal (7% fat, 300 calories) and approximately 17- and 10-fold higher, respectively, when Zytiga^{®} was administered with a high-fat (57% fat, 825 calories) meal. Disclosed in the United Kingdom Committee for Medicinal Products for Human Use (CHMP) TABLE OF CONTENTS 1. Background information", JANSSEN-CILAG INTERNATIONAL N.V., (2011-01-01): (page 11, paragraphs 2, 5; page 8, last paragraph): Zytiga compositions comprising 1. Abiraterone acetate as active substance combined with 2. lactose monohydrate, microcrystalline cellulose, croscarmellose sodium, 3. povidone, sodium lauryl sulfate, magnesium stearate and colloidal silicon dioxide. Preparation is done by (1) mixing, (2) granulation, (3) Wet milling (4) drying, (5) Dry milling, (6) Blending, (7) Lubrication (8) tablet compression and (9) packaging.

### Summary

The present disclosure features pharmaceutical compositions, including unit dosage forms, comprising fine particle abiraterone acetate as defined by the claims and also discloses but not claims methods for producing and using such compositions.

In various embodiments, the particles of abiraterone acetate in the pharmaceutical compositions have a median particle size, determined on a particle volume basis ([D₅₀] or D_{[50]} or [D50]) , which is defined by the claims and is less than a size selected from the group consisting of: 1000nm, 900nm, 800nm, 700nm, 600nm, 500nm, 400 nm, 300nm, 200nm The D[₅₀] is greater than 100nm. In various embodiments the [D50] is between: 1000nm and 100nm, 800nm and 100nm, 700nm and 100nm, 600nm and 100nm, 500nm and 100nm. The D_{[4,3]} (volume mean diameter) in various embodiments is between: 1000nm and 25nm, 1000nm and 100nm, 800nm and 100nm, 700nm and 100nm, 600nm and 100nm, 500nm and 100nm 1000nm and 200nm, 900nm and 200nm, 800nm and 200nm, 700nm and 200nm. The [D90] ([D₉₀] or D_{[90]}) in various embodiments is between: 1000nm and 300nm, 1000nm and 400nm, 1000nm and 500nm, 1000nm and 600nm, 1000nm and 700nm or 1000nm and 800nm.

In the various embodiments described herein the [D90] is less than 1000nm or less than 900nm. In some embodiments the [D₉₀] is 1000nm - 600nm, 900nm-600nm, 900nm-700nm or 900nm and 800nm.

In another embodiment, the crystallinity profile of the abiraterone acetate is selected from the group consisting of: at least 50% of the abiraterone acetate is crystalline, at least 60% of the abiraterone acetate is crystalline, at least 70% of the abiraterone acetate is crystalline, at least 75% of the abiraterone acetate is crystalline, at least 85% of the abiraterone acetate is crystalline, at least 90% of the abiraterone acetate is crystalline, at least 95% of the abiraterone acetate is crystalline and at least 98% of the abiraterone acetate is crystalline. In some embodiments, the crystallinity profile of the abiraterone acetate is substantially equal to the crystallinity profile of the abiraterone acetate before the material was subjected to the method as described herein.

In another embodiment, the amorphous content of the abiraterone acetate is selected from the group consisting of: less than 50% of the abiraterone acetate is amorphous, less than 40% of the abiraterone acetate is amorphous, less than 30% of the abiraterone acetate is amorphous, less than 25% of the abiraterone acetate is amorphous, less than 15% of the abiraterone acetate is amorphous, less than 10% of the abiraterone acetate is amorphous, less than 5% of the abiraterone acetate is amorphous and less than 2% of the abiraterone acetate is amorphous. In some embodiments, the abiraterone acetate has no significant increase in amorphous content after subjecting the material to the dry milling method described herein.

In some embodiments, the nanoparticles of abiraterone acetate are prepared by dry milling abiraterone acetate with a millable grinding compound and a facilitating agent. Additional components can be present during the milling and together the various components present during milling (with the exception of abiraterone acetate and the milling bodies) are referred to as a grinding matrix. The milling produces particles of abiraterone acetate that are significantly reduced in size dispersed in grinding matrix. Because all of the components in the grinding matrix are pharmaceutically acceptable, pharmaceutical compositions can be prepared using the mixture of abiraterone acetate and grinding matrix produced by the milling. In some cases additional pharmaceutically acceptable components can be added to the mixture of abiraterone acetate and grinding matrix. In some embodiments the dry milling takes place in the presence of milling bodies.

According to the invention abiraterone acetate is milled with one or more millable grinding compounds selected from lactose monohydrate or lactose anhydrous and mannitol and one or more facilitating agents selected from sodium lauryl sulfate and povidone as defined by the claims. In some cases abiraterone acetate is milled with lactose monohydrate and sodium lauryl sulfate.

In some cases during dry milling the abiraterone acetate can be present at 20-60% (w/w) the lactose at up to 80% (w/w) the mannitol at up to 80% (w/w) and the povidone and sodium lauryl sulfate each (or both) at 1-3% (w/w).

In some embodiments, the abiraterone acetate is dry milled in the presence of one or more antioxidants and/or one or more sequestering agents as defined by the claims, (i.e., an agent that can sequester ions, e.g, metal ions) in addition to at least one millable grinding compound and at least one facilitating agent. Thus, one or more of: butylated hydroxyanisole (BHA), butylated hydroxytoluene (BHT), ascorbic acid, fumaric acid, tartaric acid and citric acid (e.g, anhydrous citric acid) or mixtures thereof can be present during the dry milling. In some cases, both at least one antioxidant and at least one sequestering agent are present during milling. During milling, the ascorbic acid, fumaric acid, tartaric acid and citric acid (e.g, anhydrous citric acid) can be present at 8% or less on a w/w basis (e.g, 5%-0.1%, 1%-0.1%, or 0.2% each or in combination) and the BHT and BHA can be present at 0.5% or less (e.g., 0.5% - 0.01%, 0.1% - 0.08%, 0.08%-0.04%, or 0.05% each or in combination). One or more additional antioxidants and/or one or more additional sequestering agents can be added to the milled material after milling is completed.

The pharmaceutical composition can be a unit dosage form such as a capsule or tablet containing 10 - 400 mg of abiraterone acetate (e.g, 10, 15, 20, 25, 30, 35, 40, 45, 50, 55, 60, 65, 70, 75, 80, 85, 90, 95, 100, 125, 150, 175, 200, 225, 250, 275, 300, 325, 350, 375 or 400 mg).

Also described herein is a method for treating a patient comprising administering a daily dose of 900mg to 50mg of abiraterone acetate (e.g, 900, 850, 800, 750, 700, 650, 600, 550, 500, 450, 400, 350, 300, 250, 225, 200, 150, 100, 90, 80, 70, 60, 50mg) in the form of a pharmaceutical composition described herein (e.g, by administering one or more units of a unit dosage form described herein comprising abiraterone acetate). The patient can also be treated with a glucocorticoid such as prednisone, prednisolone, or dexamethasone. Alternatively, the patient can be treated with methylprednisolone (e,g., at 100 mg/day).

The references to methods of treatment in this description are to be interpreted as references to the compounds, pharmaceutical compositions and medicaments of the present invention for use in a method for treatment of the human (or animal) body by therapy.

In some cases, for the dosage forms described herein, the AUC _{0-∞} for a single dose of a dosage form described herein when administered with a low-fat meal (7% fat, 300 calories) is 4-fold or less (3-fold or less, 2-fold or less, 1.5-fold or less) higher than when administered in the fasted state.

In some cases, for the dosage forms described herein, the AUC _{0-∞} for a single dose of a dosage form described herein when administered with a high-fat meal (57% fat, 825 calories) is 8-fold or less (7-fold or less, 5-fold or less, 3-fold or less, 2-fold or less, 1.5-fold or less) higher than when administered in the fasted state.

In some cases, for the dosage forms described herein, the variation in Cmax for a single dose of a dosage form described herein when administered with a low-fat meal (7% fat, 300 calories) is 6-fold or less (5-fold or less or 4-fold or less, 3-fold or less, 2-fold or less, 1.5-fold or less) higher than when administered in the fasted state.

In some cases, for a single dose of a dosage form described herein, the variation in Cmax when administered with a high-fat meal (57% fat, 825 calories) is 15-fold or less (13-fold or less, 11-fold or less, 9-fold or less or 7-fold or less, 5-fold or less, 3-fold or less, 2-fold or less, 1.5-fold or less) higher than when administered in the fasted state.

The dissolution rate of a tablet containing 100mg of abiraterone acetate (or a 100mg of abiraterone acetate portion of a tablet containing more than 100mg of abiraterone acetate, e.g., half of a 200mg containing tablet), when tested in 900 ml of pH 4.5 phosphate buffer (0.1% SLS) using USP Apparatus II at 75 rpm is such that at least 90% or at least 95% of the abiraterone acetate dissolves in 20 min or less (e.g, 19 min or less, 18 min or less, 17 min or less, 16 min or less, 15 min or less, 14 min or less, 13 min or less, 11 min or less, 9 min or less). In cases where the tablet contains more or less than 100mg of abiraterone acetate the dissolution rate given is for a fraction of a larger tablet (or multiple of a smaller tablet) providing 100mg of abiraterone acetate).

In some cases, at least 90% or at least 95% of the abiraterone acetate dissolves in 20 min or less (e.g, 19 min or less, 18 min or less, 17 min or less, 16 min or less, 15 min or less, 14 min or less, 13 min or less, 11 min or less, 9 min or less) after storage at 4 weeks or more (e.g., 8 weeks or 12 weeks) at 25°C at 60% RH. In some cases, at least 95% of the abiraterone acetate dissolves in 15 min or less (e.g, 14 min or less, 13 min or less, 11 min or less, 9 min or less) after storage at 3 weeks or more (e.g., 6 weeks or 9 weeks) at 40°C at 75% RH. Here too, in cases where the tablet contains more or less than 100mg of abiraterone acetate the dissolution rate given is for a fraction of a larger tablet (or multiple of a smaller tablet) providing 100mg of abiraterone acetate).

In certain embodiments, in comparative pharmacokinetic testing with Zytiga^{®}, an abiraterone acetate composition of the disclosure exhibits less variability than the conventional composition. Thus, in some embodiments, the coefficient of variation observed for a pharmaceutical composition described herein in one or more of Cmax, AUC(0-t), and AUC(0-∞) will be less than 50%, less than 40%, less than 30%, less than 25%, or less than 20%. In some embodiments, a pharmaceutical composition described herein shows less variability in the average plasma concentration at any given time point after administration relative to e.g., Zytiga^{®}.

In some cases, the hardness of abiraterone tablets is between 100N and 170N (e.g., 110N to 160N).

In some embodiments, the dry milling apparatus is a mill selected from the group consisting of: attritor mills (horizontal or vertical), nutating mills, tower mills, pearl mills, planetary mills, vibratory mills, eccentric vibratory mills, gravity-dependent-type ball mills, rod mills, roller mills and crusher mills. In some embodiments, the milling bodies within the milling apparatus are mechanically agitated by 1, 2 or 3 rotating shafts. Preferably, the method is configured to produce the abiraterone acetate in a continuous fashion. The milling bodies can be formed of a material selected from the group consisting of: ceramics, glasses, steels, polymers, ferromagnetics and metals and other suitable materials. In some embodiments, the milling bodies are steel balls having a diameter selected from the group consisting of: between 1 and 20 mm, between 2 and 15 mm and between 3 and 10 mm. In various embodiments of the dry milling method, the milling bodies are zirconium oxide balls having a diameter selected from the group consisting of: between 1 and 20 mm, between 2 and 15 mm and between 3 and 10 mm. In another embodiment, the milling time period is a range selected from the group consisting of: between 10 minutes and 2 hours, between 10 minutes and 90 minutes, between 10 minutes and 1 hour, between 10 minutes and 45 minutes, between 10 minutes and 30 minutes, between 5 minutes and 30 minutes, between 5 minutes and 20 minutes, between 2 minutes and 10 minutes, between 2 minutes and 5 minutes, between 1 minutes and 20 minutes, between 1 minute and 10 minutes, and between 1 minute and 5 minutes.

### Additional Milling Matrixes and Facilitating Agents

In another embodiment, the grinding matrix is a single material or is a mixture of two or more materials in any proportion. In some embodiments, the single material or a mixture of two or more materials is selected from the group consisting of: mannitol, sorbitol, Isomalt, xylitol, maltitol, lactitol, erythritol, arabitol, ribitol, glucose, fructose, mannose, galactose, anhydrous lactose, lactose monohydrate, sucrose, maltose, trehalose, and maltodextrins. In some embodiments the single material or mixture of two or more materials is selected from the group consisting of: dextrin, Inulin, dextrates, polydextrose, starch, wheat flour, corn flour, rice flour, rice starch, tapioca flour, tapioca starch, potato flour, potato starch, other flours and starches, milk powder, skim milk powders, other milk solids and derivatives, soy flour, soy meal or other soy products, cellulose, microcrystalline cellulose, microcrystalline cellulose based co-blended materials, pregelatinized (or partially gelatinized) starch, HPMC, CMC, HPC, citric acid, tartaric acid, malic acid, maleic acid, fumaric acid, ascorbic acid, succinic acid, sodium citrate, sodium tartrate, sodium malate, sodium ascorbate, potassium citrate, potassium tartrate, potassium malate, sodium acetate, potassium ascorbate, sodium carbonate, potassium carbonate, magnesium carbonate, sodium bicarbonate, potassium bicarbonate, calcium carbonate, dibasic calcium phosphate, tribasic calcium phosphate, sodium sulfate, sodium chloride, sodium metabisulphite, sodium thiosulfate, ammonium chloride, glauber's salt, ammonium carbonate, sodium bisulfate, magnesium sulfate, potash alum, potassium chloride, sodium hydrogen sulfate, sodium hydroxide, crystalline hydroxides, hydrogen carbonates, ammonium chloride, methylamine hydrochloride, ammonium bromide, silica, thermal silica, alumina, titanium dioxide, talc, chalk, mica, kaolin, bentonite, hectorite, magnesium trisilicate, clay based materials or aluminium silicates, sodium lauryl sulfate, sodium stearyl sulfate, sodium cetyl sulfate, sodium cetostearyl sulfate, sodium docusate, sodium deoxycholate, N-lauroylsarcosine sodium salt, glyceryl monostearate, glycerol distearate glyceryl palmitostearate, glyceryl behenate, glyceryl caprylate, glyceryl oleate, benzalkonium chloride, CTAB, CTAC, Cetrimide, cetylpyridinium chloride, cetylpyridinium bromide, benzethonium chloride, PEG 40 stearate, PEG 100 stearate, poloxamer 188,poloxamer 338, poloxamer 407 polyoxyl 2 stearyl ether, polyoxyl 100 stearyl ether, polyoxyl 20 stearyl ether, polyoxyl 10 stearyl ether, polyoxyl 20 cetyl ether, polysorbate 20, polysorbate 40, polysorbate 60, polysorbate 61, polysorbate 65, polysorbate 80, polyoxyl 35 castor oil, polyoxyl 40 castor oil, polyoxyl 60 castor oil, polyoxyl 100 castor oil, polyoxyl 200 castor oil, polyoxyl 40 hydrogenated castor oil, polyoxyl 60 hydrogenated castor oil, polyoxyl 100 hydrogenated castor oil, polyoxyl 200 hydrogenated castor oil, cetostearyl alcohol, macrogel 15 hydroxystearate, sorbitan monopalmitate, sorbitan monostearate, sorbitan trioleate, sucrose palmitate, sucrose stearate, sucrose distearate, sucrose laurate, glycocholic acid, sodium glycholate, cholic acid, sodium cholate, sodium deoxycholate, deoxycholic acid, sodium taurocholate, taurocholic acid, sodium taurodeoxycholate, taurodeoxycholic acid, soy lecithin, phosphatidylcholine, phosphatidylethanolamine, phosphatidylserine, phosphatidylinositol, PEG4000, PEG6000, PEG8000, PEG10000, PEG20000, alkyl naphthalene sulfonate condensate/Lignosulfonate blend, calcium dodecylbenzene sulfonate, sodium dodecylbenzene sulfonate, diisopropyl naphthaenesulphonate, erythritol distearate, naphthalene sulfonate formaldehyde condensate, nonylphenol ethoxylate (poe-30), tristyrylphenol ethoxylate, polyoxyethylene (15) tallowalkylamines, sodium alkyl naphthalene sulfonate, sodium alkyl naphthalene sulfonate condensate, sodium alkylbenzene sulfonate, sodium isopropyl naphthalene sulfonate, sodium methyl naphthalene formaldehyde sulfonate, sodium n-butyl naphthalene sulfonate, tridecyl alcohol ethoxylate (poe-18), triethanolamine isodecanol phosphate ester, triethanolamine tristyrylphosphate ester, tristyrylphenol ethoxylate sulfate, bis(2-hydroxyethyl)tallowalkylamines.

In some embodiments, the concentration of the single (or first) grinding matrix is selected from the group consisting of: 5 - 99 % w/w, 10 - 95 % w/w, 15 - 85 % w/w, of 20 - 80% w/w, 25 - 75 % w/w, 30 - 60% w/w, 40 -50% w/w. In some embodiments, the concentration of the second or subsequent grinding matrix is selected from the group consisting of: 5 - 50 % w/w, 5 - 40 % w/w, 5 - 30 % w/w, of 5 - 20% w/w, 10 - 40 % w/w, 10 -30% w/w, 10 -20% w/w, 20 - 40% w/w, or 20 - 30% w/w or if the second or subsequent material is a surfactant or water soluble polymer the concentration is selected from 0.1 -10 % w/w, 0.1 -5 % w/w, 0.1 -2.5 % w/w, of 0.1 - 2% w/w, 0.1 -1 %, 0.5 -5% w/w, 0.5 -3% w/w, 0.5 -2% w/w, 0.5 - 1.5%, 0.5 -1 % w/w, of 0.75 - 1.25 % w/w, 0.75 -1% and 1% w/w.

In some embodiments, abiraterone acetate is milled in the presence of one or more of lactose monohydrate, lactose anhydrous and mannitol according to the claims;
(a) lactose monohydrate or lactose monohydrate combined with at least one material selected from the group consisting of: xylitol; lactose anhydrous; microcrystalline cellulose; sucrose; glucose; sodium chloride; talc; kaolin; calcium carbonate; malic acid; trisodium citrate dihydrate; D,L-Malic acid; sodium pentane sulfate; sodium octadecyl sulfate; Brij 700; Brij 76; sodium n-lauroyl sacrosine; lecithin; docusate sodium; polyoxyl-40-stearate; Aerosil R972 fumed silica; sodium lauryl sulfate or other alkyl sulfate surfactants with a chain length between C5 to C18; polyvinyl pyrrolidone;; sodium lauryl sulfate and polyethylene glycol 40 stearate, sodium lauryl sulfate and polyethylene glycol 100 stearate, sodium lauryl sulfate and PEG 3000, sodium lauryl sulphate and PEG 6000, sodium lauryl sulphate and PEG 8000, sodium lauryl sulphate and PEG 10000, sodium lauryl sulfate and Brij Brij 76700, sodium lauryl sulfate and Poloxamer 407, sodium lauryl sulfate and Poloxamer 338, sodium lauryl sulfate and Poloxamer 188; Poloxamer 407, Poloxamer 338, Poloxamer 188, alkyl naphthalene sulfonate condensate/Lignosulfonate blend; Calcium Dodecylbenzene Sulfonate (Branched); Diisopropyl naphthalenesulphonate; erythritol distearate; linear and branched dodecylbenzene sulfonic acids; Naphthalene Sulfonate Formaldehyde Condensate; nonylphenol ethoxylate, POE-30; Phosphate Esters, Tristyrylphenol Ethoxylate, Free Acid; Polyoxyethylene (15) tallowalkylamines; sodium alkyl naphthalene sulfonate; sodium alkyl naphthalene sulfonate condensate; sodium alkylbenzene sulfonate; sodium isopropyl naphthalene sulfonate; Sodium Methyl Naphthalene; Formaldehyde Sulfonate; sodium salt of n-butyl naphthalene sulfonate; tridecyl alcohol ethoxylate, POE-18; Triethanolamine isodecanol phosphate ester; Triethanolamine tristyrylphosphate ester; Tristyrylphenol Ethoxylate Sulfate; Bis(2-hydroxyethyl)tallowalkylamines.
(b) lactose anhydrous or lactose anhydrous combined with at least one material selected from the group consisting of: lactose monohydrate; xylitol; microcrystalline cellulose; sucrose; glucose; sodium chloride; talc; kaolin; calcium carbonate; malic acid; trisodium citrate dihydrate; D,L-Malic acid; sodium pentane sulfate; sodium octadecyl sulfate; Brij 700; Brij 76; sodium n-lauroyl sacrosine; lecithin; docusate sodium; polyoxyl-40-stearate; Aerosil R972 fumed silica; sodium lauryl sulfate or other alkyl sulfate surfactants with a chain length between C5 to C18; polyvinyl pyrrolidone; sodium lauryl sulfate and polyethylene glycol 40 stearate, sodium lauryl sulfate and polyethylene glycol 100 stearate, sodium lauryl sulfate and PEG 3000, sodium lauryl sulphate and PEG 6000, sodium lauryl sulphate and PEG 8000, sodium lauryl sulphate and PEG 10000, sodium lauryl sulfate and Brij 700, sodium lauryl sulfate and Poloxamer 407, sodium lauryl sulfate and Poloxamer 338, sodium lauryl sulfate and Poloxamer 188; Poloxamer 407, Poloxamer 338, Poloxamer 188, alkyl naphthalene sulfonate condensate/Lignosulfonate blend; Calcium Dodecylbenzene Sulfonate (Branched); Diisopropyl naphthalenesulphonate; erythritol distearate; linear and branched dodecylbenzene sulfonic acids; Naphthalene Sulfonate Formaldehyde Condensate; nonylphenol ethoxylate, POE-30; Phosphate Esters, Tristyrylphenol Ethoxylate, Free Acid; Polyoxyethylene (15) tallowalkylamines; sodium alkyl naphthalene sulfonate; sodium alkyl naphthalene sulfonate condensate; sodium alkylbenzene sulfonate; sodium isopropyl naphthalene sulfonate; Sodium Methyl Naphthalene; Formaldehyde Sulfonate; sodium salt of n-butyl naphthalene sulfonate; tridecyl alcohol ethoxylate, POE-18; Triethanolamine isodecanol phosphate ester; Triethanolamine tristyrylphosphate ester; Tristyrylphenol Ethoxylate Sulfate; Bis(2-hydroxyethyl)tallowalkylamines.
(c) mannitol or mannitol combined with at least one material selected from the group consisting of: lactose monohydrate; xylitol; lactose anhydrous; microcrystalline cellulose; sucrose; glucose; sodium chloride; talc; kaolin; calcium carbonate; malic acid; trisodium citrate dihydrate; D,L-Malic acid; sodium pentane sulfate; sodium octadecyl sulfate; Brij 700; Brij76; sodium n-lauroyl sacrosine; lecithin; docusate sodium; polyoxyl-40-stearate; Aerosil R972 fumed silica; sodium lauryl sulfate or other alkyl sulfate surfactants with a chain length between C5 to C18; polyvinyl pyrrolidone; sodium lauryl sulfate and polyethylene glycol 40 stearate, sodium lauryl sulfate and polyethylene glycol 100 stearate, sodium lauryl sulfate and PEG 3000, sodium lauryl sulphate and PEG 6000, sodium lauryl sulphate and PEG 8000, sodium lauryl sulphate and PEG 10000, sodium lauryl sulfate and Brij 700, sodium lauryl sulfate and Poloxamer 407, sodium lauryl sulfate and Poloxamer 338, sodium lauryl sulfate and Poloxamer 188; Poloxamer 407, Poloxamer 338, Poloxamer 188, alkyl naphthalene sulfonate condensate/Lignosulfonate blend; Calcium Dodecylbenzene Sulfonate (Branched); Diisopropyl naphthalenesulphonate; erythritol distearate; linear and branched dodecylbenzene sulfonic acids; Naphthalene Sulfonate Formaldehyde Condensate; nonylphenol ethoxylate, POE-30; Phosphate Esters, Tristyrylphenol Ethoxylate, Free Acid; Polyoxyethylene (15) tallowalkylamines; sodium alkyl naphthalene sulfonate; sodium alkyl naphthalene sulfonate condensate; sodium alkylbenzene sulfonate; sodium isopropyl naphthalene sulfonate; Sodium Methyl Naphthalene; Formaldehyde Sulfonate; sodium salt of n-butyl naphthalene sulfonate; tridecyl alcohol ethoxylate, POE-18; Triethanolamine isodecanol phosphate ester; Triethanolamine tristyrylphosphate ester; Tristyrylphenol Ethoxylate Sulfate; Bis(2-hydroxyethyl)tallowalkylamines.
(d) Sucrose or sucrose combined with at least one material selected from the group consisting of: lactose monohydrate; lactose anhydrous; mannitol; further disclosed but not falling under the scope of the claims are combinations with microcrystalline cellulose; glucose; sodium chloride; talc; kaolin; calcium carbonate; malic acid; tartaric acid; trisodium citrate dihydrate; D,L-Malic acid; sodium pentane sulfate; sodium octadecyl sulfate; Brij 700; Brij 76; sodium n-lauroyl sacrosine; lecithin; docusate sodium; polyoxyl-40-stearate; Aerosil R972 fumed silica; sodium lauryl sulfate or other alkyl sulfate surfactants with a chain length between C5 to C18; polyvinyl pyrrolidone; sodium lauryl sulfate and polyethylene glycol 40 stearate, sodium lauryl sulfate and polyethylene glycol 100 stearate, sodium lauryl sulfate and PEG 3000, sodium lauryl sulphate and PEG 6000, sodium lauryl sulphate and PEG 8000, sodium lauryl sulphate and PEG 10000, sodium lauryl sulfate and Brij 700, sodium lauryl sulfate and Poloxamer 407, sodium lauryl sulfate and Poloxamer 338, sodium lauryl sulfate and Poloxamer 188; Poloxamer 407, Poloxamer 338, Poloxamer 188, alkyl naphthalene sulfonate condensate/Lignosulfonate blend; Calcium Dodecylbenzene Sulfonate (Branched); Diisopropyl naphthalenesulphonate; erythritol distearate; linear and branched dodecylbenzene sulfonic acids; Naphthalene Sulfonate Formaldehyde Condensate; nonylphenol ethoxylate, POE-30; Phosphate Esters, Tristyrylphenol Ethoxylate, Free Acid; Polyoxyethylene (15) tallowalkylamines; sodium alkyl naphthalene sulfonate; sodium alkyl naphthalene sulfonate condensate; sodium alkylbenzene sulfonate; sodium isopropyl naphthalene sulfonate; Sodium Methyl Naphthalene; Formaldehyde Sulfonate; sodium salt of n-butyl naphthalene sulfonate; tridecyl alcohol ethoxylate, POE-18; Triethanolamine isodecanol phosphate ester; Triethanolamine tristyrylphosphate ester; Tristyrylphenol Ethoxylate Sulfate; Bis(2-hydroxyethyl)tallowalkylamines.
(e) Glucose or glucose combined with at least one material selected from the group consisting of: lactose monohydrate; lactose anhydrous; mannitol; further disclosed but not falling under the scope of the claims are combinations with microcrystalline cellulose; sucrose; sodium chloride; talc; kaolin; calcium carbonate; malic acid; tartaric acid; trisodium citrate dihydrate; D,L-Malic acid; sodium pentane sulfate; sodium octadecyl sulfate; Brij 700; Brij 76; sodium n-lauroyl sacrosine; lecithin; docusate sodium; polyoxyl-40-stearate; Aerosil R972 fumed silica; sodium lauryl sulfate or other alkyl sulfate surfactants with a chain length between C5 to C18; polyvinyl pyrrolidone; sodium lauryl sulfate and polyethylene glycol 40 stearate, sodium lauryl sulfate and polyethylene glycol 100 stearate, sodium lauryl sulfate and PEG 3000, sodium lauryl sulphate and PEG 6000, sodium lauryl sulphate and PEG 8000, sodium lauryl sulphate and PEG 10000, sodium lauryl sulfate and Brij 700, sodium lauryl sulfate and Poloxamer 407, sodium lauryl sulfate and Poloxamer 338, sodium lauryl sulfate and Poloxamer 188; Poloxamer 407, Poloxamer 338, Poloxamer 188, alkyl naphthalene sulfonate condensate/Lignosulfonate blend; Calcium Dodecylbenzene Sulfonate (Branched); Diisopropyl naphthalenesulphonate; erythritol distearate; linear and branched dodecylbenzene sulfonic acids; Naphthalene Sulfonate Formaldehyde Condensate; nonylphenol ethoxylate, POE-30; Phosphate Esters, Tristyrylphenol Ethoxylate, Free Acid; Polyoxyethylene (15) tallowalkylamines; sodium alkyl naphthalene sulfonate; sodium alkyl naphthalene sulfonate condensate; sodium alkylbenzene sulfonate; sodium isopropyl naphthalene sulfonate; Sodium Methyl Naphthalene; Formaldehyde Sulfonate; sodium salt of n-butyl naphthalene sulfonate; tridecyl alcohol ethoxylate, POE-18; Triethanolamine isodecanol phosphate ester; Triethanolamine tristyrylphosphate ester; Tristyrylphenol Ethoxylate Sulfate; Bis(2-hydroxyethyl)tallowalkylamines.
(f) Sodium chloride or sodium chloride combined with at least one material selected from the group consisting of: lactose monohydrate; lactose anhydrous; mannitol; further disclosed but not falling under the scope of the claims are combinations with microcrystalline cellulose; sucrose; glucose; talc; kaolin; calcium carbonate; malic acid; tartaric acid; trisodium citrate dihydrate; D,L-Malic acid; sodium pentane sulfate; sodium octadecyl sulfate; Brij 700; Brij 76; sodium n-lauroyl sacrosine; lecithin; docusate sodium; polyoxyl-40-stearate; Aerosil R972 fumed silica; sodium lauryl sulfate or other alkyl sulfate surfactants with a chain length between C5 to C18; polyvinyl pyrrolidone; sodium lauryl sulfate and polyethylene glycol 40 stearate, sodium lauryl sulfate and polyethylene glycol 100 stearate, sodium lauryl sulfate and PEG 3000, sodium lauryl sulphate and PEG 6000, sodium lauryl sulphate and PEG 8000, sodium lauryl sulphate and PEG 10000, sodium lauryl sulfate and Brij 700, sodium lauryl sulfate and Poloxamer 407, sodium lauryl sulfate and Poloxamer 338, sodium lauryl sulfate and Poloxamer 188; Poloxamer 407, Poloxamer 338, Poloxamer 188, alkyl naphthalene sulfonate condensate/Lignosulfonate blend; Calcium Dodecylbenzene Sulfonate (Branched); Diisopropyl naphthalenesulphonate; erythritol distearate; linear and branched dodecylbenzene sulfonic acids; Naphthalene Sulfonate Formaldehyde Condensate; nonylphenol ethoxylate, POE-30; Phosphate Esters, Tristyrylphenol Ethoxylate, Free Acid; Polyoxyethylene (15) tallowalkylamines; sodium alkyl naphthalene sulfonate; sodium alkyl naphthalene sulfonate condensate; sodium alkylbenzene sulfonate; sodium isopropyl naphthalene sulfonate; Sodium Methyl Naphthalene; Formaldehyde Sulfonate; sodium salt of n-butyl naphthalene sulfonate; tridecyl alcohol ethoxylate, POE-18; Triethanolamine isodecanol phosphate ester; Triethanolamine tristyrylphosphate ester; Tristyrylphenol Ethoxylate Sulfate; Bis(2-hydroxyethyl)tallowalkylamines.
(g) xylitol or xylitol combined with at least one material selected from the group consisting of: lactose monohydrate; lactose anhydrous; mannitol; further disclosed but not falling under the scope of the claims are combinations with microcrystalline cellulose; sucrose; glucose; sodium chloride; talc; kaolin; calcium carbonate; malic acid; tartaric acid; trisodium citrate dihydrate; D,L-Malic acid; sodium pentane sulfate; sodium octadecyl sulfate; Brij 700; Brij 76; sodium n-lauroyl sacrosine; lecithin; docusate sodium; polyoxyl-40-stearate; Aerosil R972 fumed silica; sodium lauryl sulfate or other alkyl sulfate surfactants with a chain length between C5 to C18; polyvinyl pyrrolidone; sodium lauryl sulfate and polyethylene glycol 40 stearate, sodium lauryl sulfate and polyethylene glycol 100 stearate, sodium lauryl sulfate and PEG 3000, sodium lauryl sulphate and PEG 6000, sodium lauryl sulphate and PEG 8000, sodium lauryl sulphate and PEG 10000, sodium lauryl sulfate and Brij 700, sodium lauryl sulfate and Poloxamer 407, sodium lauryl sulfate and Poloxamer 338, sodium lauryl sulfate and Poloxamer 188; Poloxamer 407, Poloxamer 338, Poloxamer 188, alkyl naphthalene sulfonate condensate/Lignosulfonate blend; Calcium Dodecylbenzene Sulfonate (Branched); Diisopropyl naphthalenesulphonate; erythritol distearate; linear and branched dodecylbenzene sulfonic acids; Naphthalene Sulfonate Formaldehyde Condensate; nonylphenol ethoxylate, POE-30; Phosphate Esters, Tristyrylphenol Ethoxylate, Free Acid; Polyoxyethylene (15) tallowalkylamines; sodium alkyl naphthalene sulfonate; sodium alkyl naphthalene sulfonate condensate; sodium alkylbenzene sulfonate; sodium isopropyl naphthalene sulfonate; Sodium Methyl Naphthalene; Formaldehyde Sulfonate; sodium salt of n-butyl naphthalene sulfonate; tridecyl alcohol ethoxylate, POE-18; Triethanolamine isodecanol phosphate ester; Triethanolamine tristyrylphosphate ester; Tristyrylphenol Ethoxylate Sulfate; Bis(2-hydroxyethyl)tallowalkylamines.
(h) Tartaric acid or tartaric acid combined with at least one material selected from the group consisting of: lactose monohydrate; lactose anhydrous; mannitol; further disclosed but not falling under the scope of the claims are combinations with microcrystalline cellulose; sucrose; glucose; sodium chloride; talc; kaolin; calcium carbonate; malic acid; trisodium citrate dihydrate; D,L-Malic acid; sodium pentane sulfate; sodium octadecyl sulfate; Brij 700; Brij 76; sodium n-lauroyl sacrosine; lecithin; docusate sodium; polyoxyl-40-stearate; Aerosil R972 fumed silica; sodium lauryl sulfate or other alkyl sulfate surfactants with a chain length between C5 to C18; polyvinyl pyrrolidone; sodium lauryl sulfate and polyethylene glycol 40 stearate, sodium lauryl sulfate and polyethylene glycol 100 stearate, sodium lauryl sulfate and PEG 3000, sodium lauryl sulphate and PEG 6000, sodium lauryl sulphate and PEG 8000, sodium lauryl sulphate and PEG 10000, sodium lauryl sulfate and Brij 700, sodium lauryl sulfate and Poloxamer 407, sodium lauryl sulfate and Poloxamer 338, sodium lauryl sulfate and Poloxamer 188; Poloxamer 407, Poloxamer 338, Poloxamer 188, alkyl naphthalene sulfonate condensate/Lignosulfonate blend; Calcium Dodecylbenzene Sulfonate (Branched); Diisopropyl naphthalenesulphonate; erythritol distearate; linear and branched dodecylbenzene sulfonic acids; Naphthalene Sulfonate Formaldehyde Condensate; nonylphenol ethoxylate, POE-30; Phosphate Esters, Tristyrylphenol Ethoxylate, Free Acid; Polyoxyethylene (15) tallowalkylamines; sodium alkyl naphthalene sulfonate; sodium alkyl naphthalene sulfonate condensate; sodium alkylbenzene sulfonate; sodium isopropyl naphthalene sulfonate; Sodium Methyl Naphthalene; Formaldehyde Sulfonate; sodium salt of n-butyl naphthalene sulfonate; tridecyl alcohol ethoxylate, POE-18; Triethanolamine isodecanol phosphate ester; Triethanolamine tristyrylphosphate ester; Tristyrylphenol Ethoxylate Sulfate; Bis(2-hydroxyethyl)tallowalkylamines.
(i) microcrystalline cellulose or microcrystalline cellulose combined with at least one material selected from the group consisting of: lactose monohydrate; lactose anhydrous; mannitol; further disclosed but not falling under the scope of the claims are combinations with xylitol; sucrose; glucose; sodium chloride; talc; kaolin; calcium carbonate; malic acid; tartaric acid; trisodium citrate dihydrate; D,L-Malic acid; sodium pentane sulfate; sodium octadecyl sulfate; Brij 700; Brij 76; sodium n-lauroyl sacrosine; lecithin; docusate sodium; polyoxyl-40-stearate; Aerosil R972 fumed silica; sodium lauryl sulfate or other alkyl sulfate surfactants with a chain length between C5 to C18; polyvinyl pyrrolidone; sodium lauryl sulfate and polyethylene glycol 40 stearate, sodium lauryl sulfate and polyethylene glycol 100 stearate, sodium lauryl sulfate and PEG 3000, sodium lauryl sulphate and PEG 6000, sodium lauryl sulphate and PEG 8000, sodium lauryl sulphate and PEG 10000, sodium lauryl sulfate and Brij 700, sodium lauryl sulfate and Poloxamer 407, sodium lauryl sulfate and Poloxamer 338, sodium lauryl sulfate and Poloxamer 188; Poloxamer 407, Poloxamer 338, Poloxamer 188, alkyl naphthalene sulfonate condensate/Lignosulfonate blend; Calcium Dodecylbenzene Sulfonate (Branched); Diisopropyl naphthalenesulphonate; erythritol distearate; linear and branched dodecylbenzene sulfonic acids; Naphthalene Sulfonate Formaldehyde Condensate; nonylphenol ethoxylate, POE-30; Phosphate Esters, Tristyrylphenol Ethoxylate, Free Acid; Polyoxyethylene (15) tallowalkylamines; sodium alkyl naphthalene sulfonate; sodium alkyl naphthalene sulfonate condensate; sodium alkylbenzene sulfonate; sodium isopropyl naphthalene sulfonate; Sodium Methyl Naphthalene; Formaldehyde Sulfonate; sodium salt of n-butyl naphthalene sulfonate; tridecyl alcohol ethoxylate, POE-18; Triethanolamine isodecanol phosphate ester; Triethanolamine tristyrylphosphate ester; Tristyrylphenol Ethoxylate Sulfate; Bis(2-hydroxyethyl)tallowalkylamines.
(j) Kaolin combined with at least one material selected from the group consisting of: lactose monohydrate;lactose anhydrous; mannitol; further disclosed but not falling under the scope of the claims are combinations with xylitol; microcrystalline cellulose; sucrose; glucose; sodium chloride; talc; kaolin; calcium carbonate; malic acid; tartaric acid; trisodium citrate dihydrate; D,L-Malic acid; sodium pentane sulfate; sodium octadecyl sulfate; Brij 700; Brij 76; sodium n-lauroyl sacrosine; lecithin; docusate sodium; polyoxyl-40-stearate; Aerosil R972 fumed silica; sodium lauryl sulfate or other alkyl sulfate surfactants with a chain length between C5 to C18; polyvinyl pyrrolidone; sodium lauryl sulfate and polyethylene glycol 40 stearate, sodium lauryl sulfate and polyethylene glycol 100 stearate, sodium lauryl sulfate and PEG 3000, sodium lauryl sulphate and PEG 6000, sodium lauryl sulphate and PEG 8000, sodium lauryl sulphate and PEG 10000, sodium lauryl sulfate and Brij 700, sodium lauryl sulfate and Poloxamer 407, sodium lauryl sulfate and Poloxamer 338, sodium lauryl sulfate and Poloxamer 188; Poloxamer 407, Poloxamer 338, Poloxamer 188, alkyl naphthalene sulfonate condensate/Lignosulfonate blend; Calcium Dodecylbenzene Sulfonate (Branched); Diisopropyl naphthalenesulphonate; erythritol distearate; linear and branched dodecylbenzene sulfonic acids; Naphthalene Sulfonate Formaldehyde Condensate; nonylphenol ethoxylate, POE-30; Phosphate Esters, Tristyrylphenol Ethoxylate, Free Acid; Polyoxyethylene (15) tallowalkylamines; sodium alkyl naphthalene sulfonate; sodium alkyl naphthalene sulfonate condensate; sodium alkylbenzene sulfonate; sodium isopropyl naphthalene sulfonate; Sodium Methyl Naphthalene; Formaldehyde Sulfonate; sodium salt of n-butyl naphthalene sulfonate; tridecyl alcohol ethoxylate, POE-18; Triethanolamine isodecanol phosphate ester; Triethanolamine tristyrylphosphate ester; Tristyrylphenol Ethoxylate Sulfate; Bis(2-hydroxyethyl)tallowalkylamines.
(k) Talc combined with at least one material selected from the group consisting of: lactose monohydrate; lactose anhydrous; mannitol; further disclosed but not falling under the scope of the claims are combinations with xylitol; microcrystalline cellulose; sucrose; glucose; sodium chloride; kaolin; calcium carbonate; malic acid; tartaric acid; trisodium citrate dihydrate; D,L-Malic acid; sodium pentane sulfate; sodium octadecyl sulfate; Brij 700; Brij 76; sodium n-lauroyl sacrosine; lecithin; docusate sodium; polyoxyl-40-stearate; Aerosil R972 fumed silica; sodium lauryl sulfate or other alkyl sulfate surfactants with a chain length between C5 to C18; polyvinyl pyrrolidone; sodium lauryl sulfate and polyethylene glycol 40 stearate, sodium lauryl sulfate and polyethylene glycol 100 stearate, sodium lauryl sulfate and PEG 3000, sodium lauryl sulphate and PEG 6000, sodium lauryl sulphate and PEG 8000, sodium lauryl sulphate and PEG 10000, sodium lauryl sulfate and Brij 700, sodium lauryl sulfate and Poloxamer 407, sodium lauryl sulfate and Poloxamer 338, sodium lauryl sulfate and Poloxamer 188; Poloxamer 407, Poloxamer 338, Poloxamer 188, alkyl naphthalene sulfonate condensate/Lignosulfonate blend; Calcium Dodecylbenzene Sulfonate (Branched); Diisopropyl naphthalenesulphonate; erythritol distearate; linear and branched dodecylbenzene sulfonic acids; Naphthalene Sulfonate Formaldehyde Condensate; nonylphenol ethoxylate, POE-30; Phosphate Esters, Tristyrylphenol Ethoxylate, Free Acid; Polyoxyethylene (15) tallowalkylamines; sodium alkyl naphthalene sulfonate; sodium alkyl naphthalene sulfonate condensate; sodium alkylbenzene sulfonate; sodium isopropyl naphthalene sulfonate; Sodium Methyl Naphthalene; Formaldehyde Sulfonate; sodium salt of n-butyl naphthalene sulfonate; tridecyl alcohol ethoxylate, POE-18; Triethanolamine isodecanol phosphate ester; Triethanolamine tristyrylphosphate ester; Tristyrylphenol Ethoxylate Sulfate; Bis(2-hydroxyethyl)tallowalkylamines.

In some embodiments, the abiraterone acetate is dry milled with one or more additional materials is selected from the group consisting of: a material considered to be `Generally Regarded as Safe' (GRAS) for pharmaceutical products.

The dry milling of abiraterone acetate takes place in the presence of a facilitating agent or combination of facilitating agents as defined by the claims. The facilitating agent is selected from the group consisting of: sodium lauryl sulfate, povidone and a stearic acid as defined by the claims.

In further disclosures, not falling under the scope of the claims, the facilitating agent is selected from the group consisting of: polyoxyethylene alkyl ethers, polyoxyethylene stearates, polyethylene glycols (PEG), poloxamers, poloxamines, sarcosine based surfactants, polysorbates, aliphatic alcohols, alkyl and aryl sulfates, alkyl and aryl polyether sulfonates and other sulfate surfactants, trimethyl ammonium based surfactants, lecithin and other phospholipids, bile salts, polyoxyethylene castor oil derivatives, polyoxyethylene sorbitan fatty acid esters, Sorbitan fatty acid esters, Sucrose fatty acid esters, alkyl glucopyranosides, alkyl maltopyranosides, glycerol fatty acid esters, Alkyl Benzene Sulphonic Acids, Alkyl Ether Carboxylic Acids, Alkyl and aryl Phosphate esters, Alkyl and aryl Sulphate esters, Alkyl and aryl Sulphonic acids, Alkyl Phenol Phosphates esters, Alkyl Phenol Sulphates esters, Alkyl and Aryl Phosphates, Alkyl Polysaccharides, Alkylamine Ethoxylates, Alkyl-Naphthalene Sulphonates formaldehyde condensates, Sulfosuccinates, lignosulfonates, Ceto-Oleyl Alcohol Ethoxylates, Condensed Naphthalene Sulphonates, Dialkyl and Alkyl Naphthalene Sulphonates, Di-alkyl Sulphosuccinates, Ethoxylated nonylphenols, Ethylene Glycol Esters, Fatty Alcohol Alkoxylates, Hydrogenated tallowalkylamines, Mono-alkyl Sulphosuccinamates, Nonyl Phenol Ethoxylates, Sodium Oleyl N-methyl Taurate, Tallowalkylamines, linear and branched dodecylbenzene sulfonic acids.

Further disclosed but not claimed are facilitating agents selected from sodium stearyl sulfate, sodium cetyl sulfate, sodium cetostearyl sulfate, sodium docusate, sodium deoxycholate, N-lauroylsarcosine sodium salt, glyceryl monostearate , glycerol distearate glyceryl palmitostearate, glyceryl behenate, glyceryl caprylate, glyceryl oleate, benzalkonium chloride, CTAB, CTAC, Cetrimide, cetylpyridinium chloride, cetylpyridinium bromide, benzethonium chloride, PEG 40 stearate, PEG 100 stearate, poloxamer 188, , poloxamer 338, poloxamer 407 polyoxyl 2 stearyl ether, polyoxyl 100 stearyl ether, polyoxyl 20 stearyl ether, polyoxyl 10 stearyl ether, polyoxyl 20 cetyl ether, polysorbate 20, polysorbate 40, polysorbate 60, polysorbate 61, polysorbate 65, polysorbate 80, polyoxyl 35 castor oil, polyoxyl 40 castor oil, polyoxyl 60 castor oil, polyoxyl 100 castor oil, polyoxyl 200 castor oil, polyoxyl 40 hydrogenated castor oil, polyoxyl 60 hydrogenated castor oil, polyoxyl 100 hydrogenated castor oil, polyoxyl 200 hydrogenated castor oil, cetostearyl alcohol, macrogel 15 hydroxystearate, sorbitan monopalmitate, sorbitan monostearate, sorbitan trioleate, Sucrose Palmitate, Sucrose Stearate, Sucrose Distearate, Sucrose laurate, Glycocholic acid, sodium Glycholate, Cholic Acid, Sodium Cholate, Sodium Deoxycholate, Deoxycholic acid, Sodium taurocholate, taurocholic acid, Sodium taurodeoxycholate, taurodeoxycholic acid, soy lecithin, phosphatidylcholine, phosphatidylethanolamine, phosphatidylserine, phosphatidylinositol, PEG4000, PEG6000, PEG8000, PEG10000, PEG20000, alkyl naphthalene sulfonate condensate/Lignosulfonate blend, Calcium Dodecylbenzene Sulfonate, Sodium Dodecylbenzene Sulfonate, Diisopropyl naphthaenesulphonate, erythritol distearate, Naphthalene Sulfonate Formaldehyde Condensate, nonylphenol ethoxylate (poe-30), Tristyrylphenol Ethoxylate, Polyoxyethylene (15) tallowalkylamines, sodium alkyl naphthalene sulfonate, sodium alkyl naphthalene sulfonate condensate, sodium alkylbenzene sulfonate, sodium isopropyl naphthalene sulfonate, Sodium Methyl Naphthalene Formaldehyde Sulfonate, sodium n-butyl naphthalene sulfonate, tridecyl alcohol ethoxylate (poe-18), Triethanolamine isodecanol phosphate ester, Triethanolamine tristyrylphosphate ester, Tristyrylphenol Ethoxylate Sulfate, Bis(2-hydroxyethyl)tallowalkylamines.

Further disclosed but not claimed are facilitating agents selected from polyvinylalcohol, acrylic acid based polymers and copolymers of acrylic acid.

In some embodiments, the facilitating agent has a concentration during dry milling selected from the group consisting of: 0.1 -10 % w/w, 0.1 -5 % w/w, 0.1 -2.5 % w/w, of 0.1 - 2% w/w, 0.1 -1 %, 0.5 -5% w/w, 0.5 -3% w/w, 0.5 -2% w/w, 0.5 - 1.5%, 0.5 -1 % w/w, of 0.75 - 1.25 % w/w, 0.75 -1% and 1% w/w.

In some embodiments, a facilitating agent is used or combination of facilitating agents is used during dry milling. In some embodiments, the facilitating agent is added during dry milling. In some embodiments, the facilitating agent is added to the dry milling at a time selected from the group consisting of: with 1-5 % of the total milling time remaining, with 1-10 % of the total milling time remaining, with 1-20 % of the total milling time remaining, with 1-30 % of the total milling time remaining, with 2-5% of the total milling time remaining, with 2-10% of the total milling time remaining, with 5-20% of the total milling time remaining and with 5-20% of the total milling time remaining.

The reasons for including facilitating agents include, but are not limited to providing better dispersibility, control of agglomeration, the release or retention of the active particles from the delivery matrix. Examples of facilitating agents include, but are not limited to: sodium lauryl sulfate, povidone and stearic acid, as defined by the claims, like povidone (PVP), Povidone K12, Povidone K17, Povidone K25, Povidone K29/32 and Povidone K30, stearic acid, magnesium stearate, calcium stearate, sodium stearyl fumarate, sodium stearyl lactylate, zinc stearate, sodium stearate or lithium stearate, while other compounds are disclosed but not claimed such as crosslinked PVP (crospovidone), cross-linked sodium carboxymethyl cellulose (croscarmellose sodium), sodium starch glycolate and other solid state fatty acids such as oleic acid, lauric acid, palmitic acid, erucic acid, behenic acid, or derivatives (such as esters and salts), Amino acids such as leucine, isoleucine, lysine, valine, methionine, phenylalanine, aspartame or acesulfame K.

In another aspect the disclosure includes a method of treating a human in need of such treatment comprising the step of administering to the human an effective amount of a pharmaceutical composition as described herein for treatment of castration resistant prostate cancer. The patient can also be treatedwith a glucocorticoid, e.g, predisone, dexamethasone or prednisolone (e.g., at 5 mg, twice daily). Alternatively, the patient can be treated with methylprednisolone. The patient can also be treated with other chemotherapeutic agents or other agents for the treatment of cancer (e.g., prostate cancer).

The disclosure also includes but not claims a method for treating breast cancer (e.g., metastatic breast cancer) and ovarian cancer (e.g., epithelial ovarian cancer) using a composition described herein.

In another aspect, the disclosure comprises the use of a pharmaceutical composition as described herein in the manufacture of a medicament for the treatment of a human in need of such treatment.

In another aspect the disclosure comprises a method for manufacturing a pharmaceutical composition as described herein comprising the step of combining a therapeutically effective amount of a composition comprising abiraterone acetate prepared by a method described herein or a composition as described herein, together with one of a diluent, lubricant, excipient, disintegrant, wetting agent, and carrier, to produce a pharmaceutically acceptable dosage form.

The disclosure described herein may include one or more ranges of values (e.g. size, concentration etc). A range of values will be understood to include all values within the range, including the values defining the range, and values adjacent to the range that lead to the same or substantially the same outcome as the values immediately adjacent to that value which defines the boundary to the range.

Throughout this specification, unless the context requires otherwise, the word "comprise" or variations, such as "comprises" or "comprising" will be understood to imply the inclusion of a stated integer, or group of integers, but not the exclusion of any other integers or group of integers. It is also noted that in this disclosure, and particularly in the claims and/or paragraphs, terms such as "comprises", "comprised", "comprising" and the like can have the meaning attributed to it in US Patent law; e.g., they can mean "includes", "included", "including", and the like.

"Therapeutically effective amount" as used herein with respect to methods of treatment and in particular drug dosage, shall mean that dosage that provides the specific pharmacological response for which the drug is administered in a significant number of subjects in need of such treatment. It is emphasized that "therapeutically effective amount," administered to a particular subject in a particular instance will not always be effective in treating the diseases described herein, even though such dosage is deemed a "therapeutically effective amount" by those skilled in the art. It is to be further understood that drug dosages are, in particular instances, measured as oral dosages, or with reference to drug levels as measured in blood.

The term "inhibit" is defined to include its generally accepted meaning which includes prohibiting, preventing, restraining, and lowering, stopping, or reversing progression or severity, and such action on a resultant symptom. As such the present disclosure includes both medical therapeutic and prophylactic administration, as appropriate.

The term "grinding matrix" is defined as any substance that a biologically active material can be or is combined with and milled the abiraterone acetate. The terms "co-grinding matrix" and "matrix" are interchangeable with "grinding matrix".

Throughout this specification, unless the context requires otherwise, the phrase "dry mill" or variations, such as "dry milling," should be understood to refer to milling in at least the substantial absence of liquids or fluids. If liquids are present, they are present in such amounts that the contents of the mill retain the characteristics of a dry powder.

"Flowable" means a powder having physical characteristics rendering it suitable for further processing using typical equipment used for the manufacture of pharmaceutical compositions and formulations.

Other definitions for selected terms used herein may be found within the detailed description of the disclosure and apply throughout. Unless otherwise defined, all other scientific and technical terms used herein have the same meaning as commonly understood to one of ordinary skill in the art to which the disclosure belongs.

The term "millable" means that the grinding matrix is capable of being reduced in size under the dry milling conditions of the method of the disclosure. In one embodiment of the disclosure, the milled grinding matrix is of a comparable particle size to the abiraterone acetate. In another embodiment of the disclosure the particle size of the matrix is substantially reduced but not as small as the abiraterone acetate

Other definitions for selected terms used herein may be found within the detailed description of the disclosure and apply throughout. Unless otherwise defined, all other scientific and technical terms used herein have the same meaning as commonly understood to one of ordinary skill in the art to which the disclosure belongs.

Other aspects and advantages of the disclosure will become apparent to those skilled in the art from a review of the ensuing description.

### Drawings

Figure 1 is a graph depicting the size distribution of abiraterone acetate in a milled arbiraterone acetate composition from Example 1 and unmilled abiraterone acetate.
Figure 2 is a graph depicting the dissolution of abiraterone acetate in a milled arbiraterone acetate composition from Example 1 and unmilled abiraterone acetate.
Figure 3 is a graph depicting the dissolution of abiraterone acetate in a milled arbiraterone acetate composition from Example 3 and unmilled abiraterone acetate.
Figure 4 is a graph depicting the size distribution of abiraterone acetate in a milled arbiraterone acetate composition from Example 1 and unmilled abiraterone acetate.
Figure 5A is a graph depicting impurities detected over time in milled DPI.
Figure 5B is a graph depicting impurities detected over time in tablets containing milled DPI
Figure 6 is graph depicting the dissolution rate of tablets containing fine powder abiraterone acetate.
Figure 7 is graph depicting the dissolution rate of a tablet containing fine powder abiraterone acetate and a tablet containing conventional abiraterone acetate.

### Detailed Description of the Disclosure

### Particle Size

There are a wide range of techniques that can be utilized to characterize the particle size of a material. Amongst these various techniques, two types of measurements are most commonly used. Photon correlation spectroscopy (PCS), also known as `dynamic light scattering' (DLS) is commonly used to measure particles with a size less than 10 micron. Typically this measurement yields an equivalent hydrodynamic radius often expressed as the average size of a number distribution. The other common particle size measurement is laser diffraction which is commonly used to measure particle size from 100 nm to 2000 micron. This technique calculates a volume distribution of equivalent spherical particles that can be expressed using descriptors such as the median particle size or the % of particles under a given size.

For measurements made using a photo correlation spectroscopy instrument, or an equivalent method known in the art, the term "number average particle size" is defined as the average particle diameter as determined on a number basis.

For measurements made using a laser diffraction the term "median particle size" is defined as the median particle diameter as determined on an equivalent spherical particle volume basis. Where the term median is used, it is understood to describe the particle size that divides the population in half such that 50 % of the population on a volume basis is greater than or less than this size. The median particle size is written as: [D₅₀] or D_{[50]} or [D50], D50, D(0.50) or D[0.5] or similar. As used herein [D₅₀] or D_{[50]} or [D50], D50, D(0.50) or D[0.5] or similar shall be taken to mean median particle size.

The term "Dx of the particle size distribution" refers to the xth percentile of the distribution on a volume basis; thus, D90 refers to the 90^{th} percentile, D95 refers to the 95^{th} percentile, and so forth. Taking D90 as an example this can often be written as, [D₉₀] or D_{[90]} or [D90], D(0.90) or D[0.9] or similar. With respect to the median particle size and Dx an upper case D or lowercase d are interchangeable and have the same meaning. Another commonly used way of describing a particle size distribution measured by laser diffraction, or an equivalent method known in the art, is to describe what % of a distribution is under or over a nominated size. The term "percentage less than" also written as "%<" is defined as the percentage, by volume, of a particle size distribution under a nominated size -for example the % < 1000 nm. The term "percentage greater than" also written as "%>" is defined as the percentage, by volume, of a particle size distribution over a nominated size -for example the % > 1000 nm.

For many of the materials subject to the methods of this disclosure the particle size can be easily measured. Where the active material has poor water solubility and the matrix it is milled in has good water solubility the powder can simply be dispersed in an aqueous solvent. In this scenario the matrix dissolves leaving the active material dispersed in the solvent. This suspension can then be measured by techniques such as PCS or laser diffraction.

Suitable methods to measure an accurate particle size where the active material has substantive aqueous solubility or the matrix has low solubility in a water based dispersant are outlined below.
1. In the circumstance where an insoluble matrix such as microcrystalline cellulose prevents the measurement of the active material separation techniques such as filtration or centrifugation could be used to separate the insoluble matrix from the active material particles. Other ancillary techniques would also be required to determine if any active material was removed by the separation technique so that this could be taken into account.
2. In the case where the active material is too soluble in water, other solvents could be evaluated for the measurement of particle size. Where a solvent could be found that active material is poorly soluble in but is a good solvent for the matrix a measurement would be relatively straight forward. If such a solvent is difficult to find another approach would be to measure the ensemble of matrix and active material in a solvent (such as iso-octane) which both are insoluble in. Then the powder would be measured in another solvent where the active material is soluble but the matrix is not. Thus with a measurement of the matrix particle size and a measurement of the size of the matrix and active material together an understanding of the active material particle size can be obtained.
3. In some circumstances image analysis could be used to obtain information about the particle size distribution of the active material. Suitable image measurement techniques might include transmission electron microscopy (TEM), scanning electron microscopy (SEM), optical microscopy and confocal microscopy. In addition to these standard techniques some additional technique would be required to be used in parallel to differentiate the active material and matrix particles. Depending on the chemical makeup of the materials involved possible techniques could be elemental analysis, Raman spectroscopy, FTIR spectroscopy or fluorescence spectroscopy.

### Improving the dissolution profile

The process results in the abiraterone acetate having an improved dissolution profile. An improved dissolution profile has significant advantages including the improvement of bioavailability of the abiraterone acetate *in vivo.* In some embodiments, the improved dissolution profile is observed *in vitro.* Alternatively, the improved dissolution profile is observed *in vivo* by the observation of an improved bioavailability profile. Standard methods for determining the dissolution profile of a material *in vitro* are available in the art. A suitable method to determine an improved dissolution profile *in vitro* may include determining the concentration of the sample material in a solution over a period of time and comparing the results from the sample materialto a control sample. An observation that peak solution concentration for the sample material was achieved in less time than the control sample would indicate (assuming it is statistically significant), that the sample material has an improved dissolution profile. The measurement sample is herein defined as the mixture of abiraterone acetate with grinding matrix and/or other additives that has been subject to the processes of the disclosure described here. Herein a control sample is defined as a physical mixture (not subject to the processes described in this disclosure) of the components in the measurement sample with the same relative proportions of active, matrix and/or additive as the measurement sample. For the purposes of the dissolution testing a prototype formulation of the measurement sample could also be used. In this case the control sample would be formulated in the same way. Standard methods for determining the improved dissolution profile of a material *in vivo* are available in the art. A suitable method to determine an improved dissolution profile in a human may be after delivering the dose to measure the rate of active material absorption by measuring the plasma concentration of the sample compound over a period of time and comparing the results from the sample compound to a control. An observation that peak plasma concentration for the sample compound was achieved in less time than the control would indicate (assuming it is statistically significant) that the sample compound has improved bioavailability and an improved dissolution profile. In some embodiments, the improved dissolution profile is observed at a relevant gastrointestinal pH, when it is observed *in vitro.* In some embodiments, the improved dissolution profile is observed at a pH which is favourable at indicating improvements in dissolution when comparing the measurement sample to the control compound. Suitable methods for quantifying the concentration of a compound in an *in vitro* sample or an *in vivo* sample are widely available in the art. Suitable methods could include the use of spectroscopy or radioisotope labeling.

### Crystallization Profile

Methods for determining the crystallinity profile of the abiraterone acetate are widely available in the art. Suitable methods may include X-ray diffraction, differential scanning calorimetry, Raman or IR spectrocopy.

### Amorphicity Profile

Methods for determining the amorphous content of the abiraterone acetate are widely available in the art. Suitable methods may include X-ray diffraction, differential scanning calorimetry, Raman or IR spectroscopy.

### Grinding Matrix

As will be described subsequently, selection of an appropriate grinding matrix affords particular advantageous applications of the method of the present disclosure.

Again, as will be described subsequently, a highly advantageous aspect of the present disclosure is that certain grinding matrixes appropriate for use in the method of the disclosure are also appropriate for use in a medicament. The present disclosure encompasses methods for the production of a medicament incorporating both the abiraterone acetate and the grinding matrix or in some cases the abiraterone acetate and a portion of the grinding matrix, medicaments so produced, and methods of treatment using the medicament. The medicament may include only the milled abiraterone acetate together with the milled grinding matrix or, more preferably, the milled abiraterone acetate and milled grinding matrix may be combined with one or more pharmaceutically acceptable carriers, as well as any desired excipients or other like agents commonly used in the preparation of medicaments.

In some cases at least one component of the grinding matrix is harder than the abiraterone acetate, and is thus capable of reducing the particle size of the abiraterone acetate under the dry milling conditions of the disclosure. Again without wishing to be bound by theory, under these circumstances it is believed that the millable grinding matrix affords the advantage of the present disclosure through a second route, with the smaller particles of grinding matrix produced under the dry milling conditions enabling greater interaction with the abiraterone acetate.

The quantity of the grinding matrix relative to the quantity of abiraterone acetate, and the extent of physical degradation of the grinding matrix, is sufficient to inhibit re-agglomeration of the particles of the active material In some embodiments, the quantity of the grinding matrix relative to the quantity of abiraterone acetate, and the extent of size reduction of the grinding matrix, is sufficient to inhibit re-agglomeration of the particles of the active material.. As detailed above, the grinding matrix can include one or more anti-oxidants and/or one or more sequestering agents.

In some embodiments, the grinding matrix has a low tendency to agglomerate during dry milling. While it is difficult to objectively quantify the tendency to agglomerate during milling, it is possible to obtain a subjective measure by observing the level of "caking" of the grinding matrix on the milling bodies and the milling chamber of the media mill as dry milling progresses.

The grinding matrix may be an inorganic or organic substance.

### Milling bodies

In the method of the present disclosure, the milling bodies are preferably chemically inert and rigid. The term "chemically-inert", as used herein, means that the milling bodies do not react chemically with the abiraterone acetate or the grinding matrix.

As described above, the milling bodies are essentially resistant to fracture and erosion in the milling process.

The milling bodies are desirably provided in the form of bodies which may have any of a variety of smooth, regular shapes, flat or curved surfaces, and lacking sharp or raised edges. For example, suitable milling bodies can be in the form of bodies having ellipsoidal, ovoid, spherical or right cylindrical shapes. In some embodiments, the milling bodies are provided in the form of one or more of beads, balls, spheres, rods, right cylinders, drums or radius-end right cylinders (i.e., right cylinders having hemispherical bases with the same radius as the cylinder).

Depending on the nature of the abiraterone acetate and the grinding matrix, the milling bodies desirably have an effective mean particle diameter (i.e. "particle size") between about 0.1 and 30 mm, more preferably between about 1 and about 15 mm, still more preferably between about 3 and 10 mm.

The milling bodies may comprise various substances such as ceramic, glass, metal or polymeric compositions, in a particulate form. Suitable metal milling bodies are typically spherical and generally have good hardness (i.e. RHC 60-70), roundness, high wear resistance, and narrow size distribution and can include, for example, balls fabricated from type 52100 chrome steel, type 304, 316 or 440C stainless steel or type 1065 high carbon steel.

Ceramics, for example, can be selected from a wide array of ceramics desirably having sufficient hardness and resistance to fracture to enable them to avoid being chipped or crushed during milling and also having sufficiently high density. Suitable densities for milling bodies can range from about 1 to 15 g/cm³, preferably from about 1 to 8 g/cm³. Ceramics can be selected from steatite, aluminum oxide, zirconium oxide, zirconia-silica, yttria-stabilized zirconium oxide, magnesia-stabilized zirconium oxide, silicon nitride, silicon carbide, cobalt-stabilized tungsten carbide, and the like, as well as mixtures thereof.

Glass milling bodies are spherical (e.g. beads), have a narrow size distribution, are durable, and include, for example, lead-free soda lime glass and borosilicate glass. Polymeric milling bodies are preferably substantially spherical and can be selected from a wide array of polymeric resins having sufficient hardness and friability to enable them to avoid being chipped or crushed during milling, abrasion-resistance to minimize attrition resulting in contamination of the product, and freedom from impurities such as metals, solvents, and residual monomers.

Milling bodies can be formed from polymeric resins. Polymeric resins, for example, can be selected from crosslinked polystyrenes, such as polystyrene crosslinked with divinylbenzene, styrene copolymers, polyacrylates such as polymethylmethacrylate, polycarbonates, polyacetals, vinyl chloride polymers and copolymers, polyurethanes, polyamides, high density polyethylenes, polypropylenes, and the like. The use of polymeric milling bodies to grind materials down to a very small particle size (as opposed to mechanochemical synthesis) is disclosed, for example, in U.S. patents 5,478,705 and 5,500,331. Polymeric resins typically can have densities ranging from about 0.8 to 3.0 g/cm³. Higher density polymeric resins are generally preferred. Alternatively, the milling bodies can be composite bodies comprising dense core bodies having a polymeric resin adhered thereon. Core particles can be selected from substances known to be useful as milling bodies, for example, glass, alumina, zirconia silica, zirconium oxide, stainless steel, and the like. Core substances have densities greater than about 2.5 g/cm³.

In one embodiment of the disclosure, the milling bodies are formed from a ferromagnetic substance, thereby facilitating removal of contaminants arising from wear of the milling bodies by the use of magnetic separation techniques.

Each type of milling body has its own advantages. For example, metals have the highest specific gravities, which increase grinding efficiency due to increased impact energy. Metal costs range from low to high, but metal contamination of final product can be an issue. Glasses are advantageous from the standpoint of low cost and the availability of small bead sizes as low as 0.004 mm. However, the specific gravity of glasses is lower than other bodies and significantly more milling time is required. Finally, ceramics are advantageous from the standpoint of low wear and contamination, ease of cleaning, and high hardness.

### Dry Milling

In the dry milling process of the present disclosure, the abiraterone acetate and grinding matrix, in the form of crystals, powders, or the like, are combined in suitable proportions with the plurality of milling bodies in a milling chamber that is mechanically agitated (i.e. with or without stirring) for a predetermined period of time at a predetermined intensity of agitation. Typically, a milling apparatus is used to impart motion to the milling bodies by the external application of agitation, whereby various translational, rotational or inversion motions or combinations thereof are applied to the milling chamber and its contents, or by the internal application of agitation through a rotating shaft terminating in a blade, propeller, impeller or paddle or by a combination of both actions.

During milling, motion imparted to the milling bodies can result in application of shearing forces as well as multiple impacts or collisions having significant intensity between milling bodies and particles of the abiraterone acetate and grinding matrix. The nature and intensity of the forces applied by the milling bodies to the abiraterone acetate and the grinding matrix is influenced by a wide variety of processing parameters including: the type of milling apparatus; the intensity of the forces generated, the kinematic aspects of the process; the size, density, shape, and composition of the milling bodies; the weight ratio of the abiraterone acetate and grinding matrix mixture to the milling bodies; the duration of milling; the physical properties of both the abiraterone acetate and the grinding matrix; the atmosphere present during activation; and others.

Advantageously, the media mill is capable of repeatedly or continuously applying mechanical compressive forces and shear stress to the abiraterone acetate and the grinding matrix. Suitable media mills include but are not limited to the following: high-energy ball, sand, bead or pearl mills, basket mill, planetary mill, vibratory action ball mill, multi-axial shaker/mixer, stirred ball mill, horizontal small media mill, multi-ring pulverizing mill, and the like, including small milling media. The milling apparatus also can contain one or more rotating shafts.

In a form of the disclosure, the dry milling is performed in a ball mill. Throughout the remainder of the specification reference will be made to dry milling being carried out by way of a ball mill. Examples of this type of mill are attritor mills, nutating mills, tower mills, planetary mills, vibratory mills and gravity-dependent-type ball mills. It will be appreciated that dry milling in accordance with the method of the disclosure may also be achieved by any suitable means other than ball milling. For example, dry milling may also be achieved using jet mills, rod mills, roller mills or crusher mills.

In some cases, the particle size of the abiraterone acetate prior to dry milling according to the methods described herein in less than about 1000 µm, as determined by sieve analysis. If the particle size of the abiraterone acetate is greater than about 1000 µm, then it is preferred that the particles of the abiraterone acetate substrate be reduced in size to less than 1000 µm using another standard milling method prior to dry milling according to the methods described herein.

### Agglomerates of abiraterone acetate after processing

Agglomerates comprising particles of abiraterone acetate having a particle size within the ranges specified herein, should be understood to fall within the scope of the present disclosure, regardless of whether the agglomerates exceed the ranges specified above.

### Processing Time

In some embodiments, the abiraterone acetate and the grinding matrix are dry milled for the shortest time necessary to minimise any possible contamination from the media mill and/or the plurality of milling bodies. This time varies greatly, depending on the abiraterone acetate and the grinding matrix, and may range from as short as 1 minute to several hours.

Suitable rates of agitation and total milling times are adjusted for the type and size of milling apparatus as well as the milling media, the weight ratio of the abiraterone acetate and grinding matrix mixture to the plurality of milling bodies, the chemical and physical properties of the abiraterone acetate and grinding matrix, and other parameters that may be optimized empirically.

In some embodiments, the grinding matrix (the materials milled together with abiraterone acetate) is not separated from the abiraterone acetate but is maintained with the abiraterone acetate in the final product. In some embodiments the grinding matrix is considered to be Generally Regarded as Safe (GRAS) for pharmaceutical products.

In an alternative aspect, the grinding matrix is separated from the abiraterone acetate. In one aspect, where the grinding matrix is not fully milled, the unmilled grinding matrix is separated from the abiraterone acetate. In a further aspect, at least a portion of the milled grinding matrix is separated from the abiraterone acetate.

Any portion of the grinding matrix may be removed, including but not limited to 10%, 25%, 50%, 75%, or substantially all of the grinding matrix.

In some embodiments of the disclosure, a significant portion of the milled grinding matrix may comprise particles of a size similar to and/or smaller than the particles comprising the abiraterone acetate. Where the portion of the milled grinding matrix to be separated from the particles comprising the abiraterone acetate comprises particles of a size similar to and/or smaller than the particles comprising the abiraterone acetate, separation techniques based on size distribution are inapplicable. In these circumstances, the method of the present disclosure may involve separation of at least a portion of the milled grinding matrix from the abiraterone acetate by techniques including but not limited to electrostatic separation, magnetic separation, centrifugation (density separation), hydrodynamic separation, and froth flotation. Advantageously, the step of removing at least a portion of the milled grinding matrix from the abiraterone acetate may be performed through means such as selective dissolution, washing, or sublimation.

An advantageous aspect of the disclosure would be the use of grinding matrix that has two or more components where at least one component is water soluble and at least one component has low solubility in water. In this case washing can be used to remove the matrix component soluble in water leaving the abiraterone acetate dispersed in the remaining matrix components. In a highly advantageous aspect of the disclosure the matrix with low solubility is a functional excipient.

A highly advantageous aspect of the present disclosure is that certain grinding matrixes appropriate for use in the method of the disclosure are also pharmaceutically acceptable and thus appropriate for use in a medicament. Where the method of the present disclosure does not involve complete separation of the grinding matrix from the abiraterone acetate, the present disclosure encompasses methods for the production of a medicament incorporating both the abiraterone acetate and at least a portion of the milled grinding matrix, medicaments so produced and methods of treatment of an animal, including man, using a therapeutically effective amount of said abiraterone acetate by way of said medicaments.

### Abiraterone acetate and compositions

The present disclosure encompasses pharmaceutically acceptable materials produced according to the methods of the present disclosure, compositions including such materials, including compositions comprising such materials together with the grinding matrix with or without milling aids, facilitating agents, as defined by the claims, with at least a portion of the grinding matrix or separated from the grinding matrix.

The pharmaceutically acceptable materials within the compositions of the disclosure are present at a concentration of between about 0.1% and about 99.0% by weight. In some embodiments, the concentration of pharmaceutically acceptable materials within the compositions will be about 5% to about 80% by weight, e.g., about 10% to about 50% by weigh. Desirably, the concentration will be in the range of about 10 to 15% by weight, 15 to 20% by weight, 20 to 25% by weight, 25 to 30% by weight, 30 to 35% by weight, 35 to 40% by weight, 40 to 45% by weight, 45 to 50% by weight, 50 to 55% by weight, 55 to 60% by weight, 60 to 65% by weight, 65 to 70% by weight, 70 to 75% by weight or 75 to 80% by weight for the composition prior to any later removal (if desired) of any portion of the grinding matrix. Where part or all of the grinding matrix has been removed, the relative concentration of pharmaceutically acceptable materials in the composition may be considerably higher depending on the amount of the grinding matrix that is removed. For example, if all of the grinding matrix is removed the concentration of particles in the preparation may approach 100% by weight (subject to the presence of facilitating agents).

### Medicaments

The medicaments of the present disclosure may include the pharmaceutically acceptable material, optionally together with the grinding matrix or at least a portion of the grinding matrix, with or without milling aids, facilitating agents, combined with one or more pharmaceutically acceptable carriers, as well as other agents commonly used in the preparation of pharmaceutically acceptable compositions.

As used herein "pharmaceutically acceptable carrier" includes any and all solvents, dispersion media, coatings, antibacterial and antifungal agents, isotonic and absorption delaying agents, and the like that are physiologically compatible. In some embodiments, the carrier is suitable for parenteral administration, intravenous, intraperitoneal, intramuscular, sublingual, pulmonary, transdermal or oral administration. Pharmaceutically acceptable carriers include sterile aqueous solutions or dispersions and sterile powders for the extemporaneous preparation of sterile injectable solutions or dispersion. The use of such media and agents for the manufacture of medicaments is well known in the art. Except insofar as any conventional media or agent is incompatible with the pharmaceutically acceptable material, use thereof in the manufacture of a pharmaceutical composition according to the disclosure is contemplated.

Pharmaceutical acceptable carriers according to the disclosure may include one or more of the following examples:
(1) surfactants and polymers including, but not limited to polyethylene glycol (PEG), polyvinylpyrrolidone (PVP), polyvinylalcohol, crospovidone, polyvinylpyrrolidone-polyvinylacrylate copolymer, cellulose derivatives, hydroxypropylmethyl cellulose, hydroxypropyl cellulose, carboxymethylethyl cellulose, hydroxypropyllmethyl cellulose phthalate, polyacrylates and polymethacrylates, urea, sugars, polyols, and their polymers, emulsifiers, sugar gum, starch, organic acids and their salts, vinyl pyrrolidone and vinyl acetate
(2) binding agents such as various celluloses and cross-linked polyvinylpyrrolidone, microcrystalline cellulose; and or
(3) filling agents such as lactose monohydrate, lactose anhydrous, microcrystalline cellulose and various starches; and or
(4) lubricating agents such as agents that act on the flowability of the powder to be compressed, including colloidal silicon dioxide, talc, stearic acid, magnesium stearate, calcium stearate, silica gel; and or
(5) sweeteners such as any natural or artificial sweetener including sucrose, xylitol, sodium saccharin, cyclamate, aspartame, and acesulfame K; and or
(6) flavouring agents; and or
(7) preservatives such as potassium sorbate, methylparaben, propylparaben, benzoic acid and its salts, other esters of parahydroxybenzoic acid such as butylparaben, alcohols such as ethyl or benzyl alcohol, phenolic chemicals such as phenol, or quarternary compounds such as benzalkonium chloride; and or
(8) buffers; and or
(9) Diluents such as pharmaceutically acceptable inert fillers, such as microcrystalline cellulose, lactose, dibasic calcium phosphate, saccharides, and/or mixtures of any of the foregoing; and or
(10) wetting agents such as corn starch, potato starch, maize starch, and modified starches, and mixtures thereof; and or
(11) disintegrants; such as croscarmellose sodium, crospovidone, sodium starch glycolate, and or
(12) effervescent agents such as effervescent couples such as an organic acid (e.g., citric, tartaric, malic, fumaric, adipic, succinic, and alginic acids and anhydrides and acid salts), or a carbonate (e.g. sodium carbonate, potassium carbonate, magnesium carbonate, sodium glycine carbonate, L-lysine carbonate, and arginine carbonate) or bicarbonate (e.g. sodium bicarbonate or potassium bicarbonate); and or
(13) other pharmaceutically acceptable excipients.

Actual dosage levels of abiraterone acetate disclosure may be varied in accordance with the nature of the abiraterone acetate, as well as the potential increased efficacy due to the advantages of providing and administering the abiraterone acetate (e.g., increased solubility, more rapid dissolution, increased surface area of the abiraterone acetate, etc.). Thus as used herein "therapeutically effective amount" will refer to an amount of abiraterone acetate required to effect a therapeutic response in an animal. Amounts effective for such a use will depend on: the desired therapeutic effect; the route of administration; the potency of the abiraterone acetate; the desired duration of treatment; the stage and severity of the disease being treated; the weight and general state of health of the patient; and the judgment of the prescribing physician.

### Pharmacokinetic Properties of Abiraterone acetate Compositions

### Fast Onset of Activity

In some embodiments, the abiraterone acetate compositions of the disclosure are rapidly absorbed. In one example, following administration the abiraterone acetate compositions of the disclosure comprising abiraterone acetate have a Tₘₐₓ of less than about 2 hours, less than about 1.75 hours, less than about 1.5 hours, less than about 1.25 hours, less than about 1.0 hours, less than about 50 minutes, less than about 40 minutes, or less than about 30 minutes.

### Increased Bioavailability

The abiraterone acetate compositions of the disclosure exhibit increased bioavailability (AUC) and require smaller doses as compared to prior conventional compositions administered at the same dose (e.g., Zytiga^{®}). Any drug composition can have adverse side effects. Thus, lower doses of drugs which can achieve the same or better therapeutic effects as those observed with larger doses of conventional compositions are desired. Such lower doses can be realized with the compositions of the disclosure because the greater bioavailability observed with the compositions as compared to conventional drug formulations means that smaller doses of drug are required to obtain the desired therapeutic effect.

### The Pharmacokinetic Profiles of the Compositions of the Disclosure are less Substantially

### Affected by the Fed or Fasted State of the Subject Ingesting the Compositions

The disclosure encompasses abiraterone acetate compositions wherein the pharmacokinetic profile of the composition is less substantially affected by the fed or fasted state of a subject ingesting the composition compared to Zytiga^{®}. This means that there is a less substantial difference in the quantity of composition or the rate of composition absorption when the compositions are administered in the fed versus the fasted state. Thus, the compositions of the disclosure substantially reduce the effect of food on the pharmacokinetics of the composition compared to Zytiga^{®}.

Any standard pharmacokinetic protocol can be used to determine blood plasma concentration profile in humans following administration of a composition, and thereby establish whether that composition meets the pharmacokinetic criteria set out herein. For example, a randomized single-dose crossover study can be performed using a group of healthy adult human subjects. The number of subjects should be sufficient to provide adequate control of variation in a statistical analysis, and is typically about 10 or greater, although for certain purposes a smaller group can suffice. Each subject receives by oral administration at time zero a single dose (e.g., 100 mg) of a test formulation of composition, normally at around 8 am following an overnight fast. The subjects continue to fast and remain in an upright position for about 4 hours after administration of the composition. Blood samples are collected from each subject prior to administration (e.g., 15 minutes) and at several intervals after administration. For the present purpose it is to take several samples within the first hour, and to sample less frequently thereafter. Illustratively, blood samples could be collected at 15, 30, 45, 60, and 90 minutes after administration, then every hour from 2 to 10 hours after administration. Additional blood samples may also be taken later, for example at 12 and 24 hours after administration. If the same subjects are to be used for study of a second test formulation, a period of at least 7 days should elapse before administration of the second formulation. Plasma is separated from the blood samples by centrifugation and the separated plasma is analyzed for composition by a validated high performance liquid chromatography (HPLC) or liquid chromatography mass spectrometry (LCMS) procedure. Plasma concentrations of composition referenced herein are intended to mean total concentrations including both free and bound composition.

Any formulation giving the desired pharmacokinetic profile is suitable for administration according to the present methods. Exemplary types of formulations giving such profiles are liquid dispersions and solid dose forms of composition. If the liquid dispersion medium is one in which the composition has very low solubility, the particles are present as suspended particles.

### Modes of administration of medicaments comprising abiraterone acetates

Medicaments of the disclosure can be administered to animals, including man, in any pharmaceutically acceptable manner, such as orally, rectally, pulmonary, intravaginally, locally (powders, ointments or drops), transdermal, parenteral administration, intravenous, intraperitoneal, intramuscular, sublingual or as a buccal or nasal spray.

Solid dosage forms for oral administration include capsules, tablets, pills, powders, pellets, and granules. Further, incorporating any of the normally employed excipients, such as those previously listed, and generally 5-95% of the biologically active agent, and more preferably at a concentration of 10%-75% will form a pharmaceutically acceptable non-toxic oral composition.

However, if the abiraterone acetate is to be utilized in a liquid suspension, the particles comprising the abiraterone acetate may require further stabilization once the solid carrier has been substantially removed to ensure the elimination, or at least minimisation of particle agglomeration.

Comparative example

### 1. Preparation of fine particle abiraterone acetate

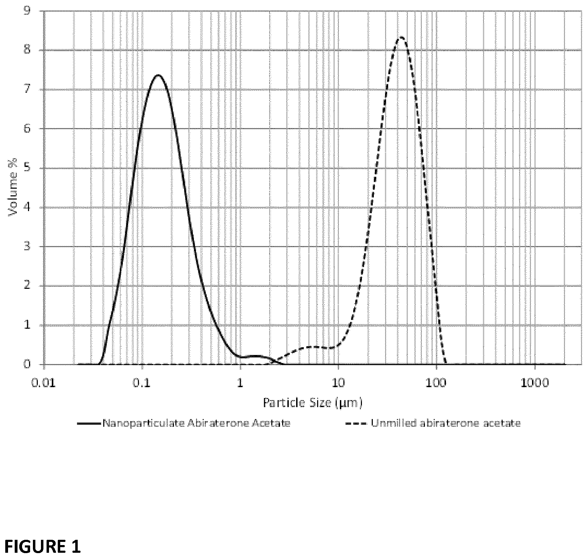

Abiraterone acetate drug substance was obtained from Hangzhao Dayangchem Co., Ltd.; Hangzhao City, P.R. China. A mixture (3.0 g) of abiraterone acetate (10% w/w), lactose monohydrate (89%; DMV Fonterra), and sodium lauryl sulfate (1%; Sigma-Aldrich) was milled in a Spex vibratory mill with milling bodies for 10 minutes to yield 2.7 g of nanoparticulate abiraterone acetate. The particle size distributions of unmilled abiraterone acetate and nanoparticulate abiraterone acetate were determined by light scattering techniques (Malvern Mastersizer 2000); the average particle size of the unmilled material was approximately 40 µm (volume statistics; D₁₀=16 µm, D₅₀=37 µm; D₉₀=70 µm) whereas the average size of the nanoparticulate abiraterone acetate drug particles was approximately 1000 nm (D₁₀=75 nm; D₅₀=177 nm; D₉₀=2.5 µm). The results are depicted graphically in Figure 1

### Example 2: Comparative Dissolution Studies

Approximately 250 mg of the nanoparticulate abiraterone acetate formulation prepared in Example 1 (corresponding to 25 mg of active ingredient) was hand-filled into size 1 hard gelatin capsules. Also, 250 mg of unmilled abiraterone acetate was hand filled into similar capsules. Dissolution profiles were measured in triplicate using a Varian VK7025 dissolution apparatus fitted with (6) 1000 mL vessels, with detection performed in a Varian UV-Vis Spectrophotometer at 270 nm. Each vessel contained 900 mL dissolution media consisting of 10 mmol phosphate buffer at pH 6 with 0.1% SDS added. Experiments were conducted at 37°C. The hard gelatin capsules were fitted inside spiral sinkers before commencing the dissolution experiments.

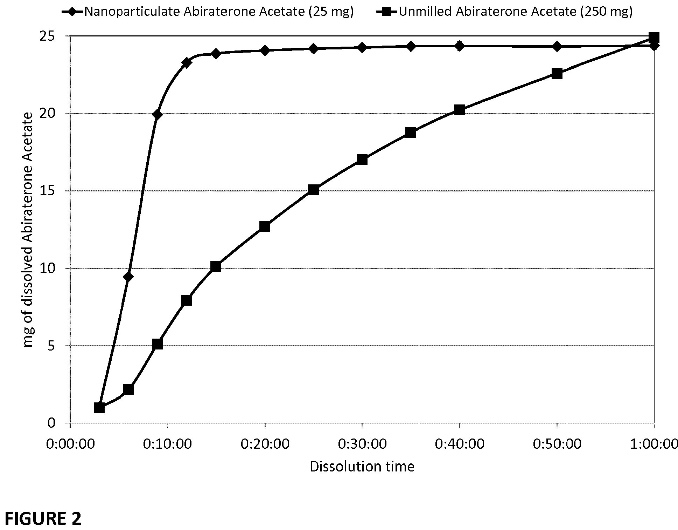

The results indicate that under the conditions studied, only approximately 10% of the unmilled abiraterone acetate (25 mg) is dissolved after one hour, while 100% of the nanoparticulate abiraterone acetate the dissolution (25 mg) is dissolved within approximately 10 minutes (Figure 2).

### Comparative example

### 3: Preparation of fine particle abiraterone acetate

Abiraterone acetate was obtained from Chongquing Pharmaceutical Research Institute (China). Lactose Monohydrate NF was obtained from Meggle Pharma (CapsuLac^{®} 60). Sodium lauryl sulfate NF was obtained from Cognis (Texapon^{®} K12 P PH). A SPEX Sample Prep 5100 Mixer Mill (Metuchen, NJ) with a 2.5 ml stainless steel grinding vial and two ¼" stainless steel grinding balls was used to prepare the nanoparticulate abiraterone acetate. In each experiment, 100 mg of pre-blended powder was added, the grinding vial was capped and the mixer mill was run for 20 minutes. The milled powder used for particle size analysis consisted of abiraterone acetate (30 mg), lactose monohydrate (68.5 mg) and sodium lauryl sulfate (1.5 mg). The milled powder used for dissolution studies consisted of abiraterone acetate (20 mg), lactose monohydrate (78.5 mg) and sodium lauryl sulfate (1.5 mg). In order to have enough material for dissolution testing, milled powder from several experiments was combined.

### Comparative example

### 4: Particle size analysis of milled and unmilled abiraterone acetate

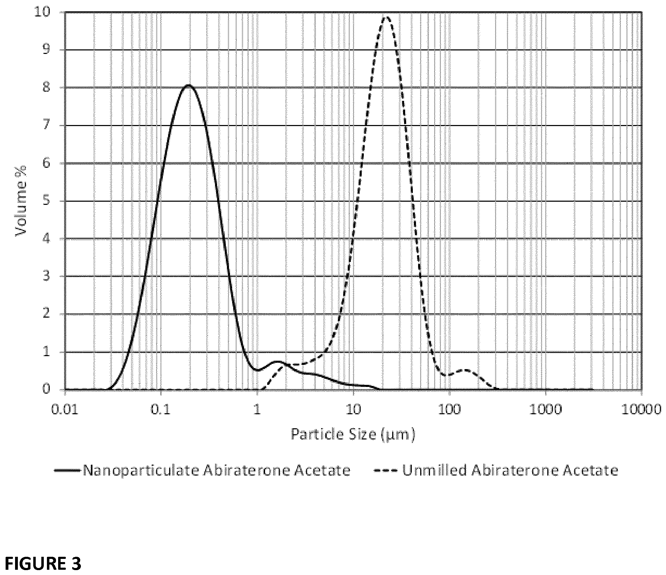

Milled powder samples from Example 3 were analyzed by adding 26 mg of milled material (6 mg of abiraterone acetate) to 5 ml of 0.1% w/w aqueous polyvinylpyrrolidone solution (PVP; BASF Kollidon^{®} 30), then sonicating with an external sonication horn (Branson Digital Sonifier^{®}) for 5 seconds at 20% amplitude followed by a 15 second pause. This cycle was continued until the total sonication time reached one minute. This suspension was then added dropwise to the sample cell of a Malvern Mastersizer 3000 particle size analyzer (Malvern Hydro MV pump unit) containing 125 ml of 0.1% PVP. The sample was then allowed to stir for 5 minutes prior to taking measurements. Data from the final measurements are presented in Table 1 and depicted graphically in Figure 3.

For comparison, the particle size distribution of unmilled abiraterone acetate (raw drug substance) was also determined. The measurement conditions were similar as described above with the exception that the unmilled abiraterone acetate was added directly to the Malvern Hydro MV pump unit containing 130 ml of 0.1% PVP. 26 mg of unmilled abiraterone acetate had to be added directly to the Malvern pump unit in order to obtain obscuration values similar to that of the milled material. The abiraterone acetate was then subjected to 1 minute of bath sonication at 100% amplitude. The sample was then allowed to stir for 5 minutes prior to taking measurements. The data from these measurements are presented in Table 1 and depicted graphically in Figure 3. The results indicate that the milled abiraterone acetate material contained fine particle drug substance and that the size of the drug particles in the milled abiraterone acetate material was substantially smaller (greater than 10 times) than in the unmilled material. No fine particle abiraterone acetate was measured in the unmilled drug sample.

### Malvern Mastersizer 3000 settings:

| | | | |
|---|---|---|---|
| Optical properties for abiraterone acetate: | | Refractive Index: | 1.583 |
| | | Absorption: | 0.01 |
| Optical properties for dispersant: | | Refractive Index: | 1.33 |
| Sample measurement time: | 10 seconds | | |
| Background measurement time: | 10 seconds | | |
| Number of measurement cycles: | 3 (Results reported are an average of these) | | |
| Delay between cycles: | 0 | | |
| Stirrer setting: | 2000 rpm | | |

**Table 1: Comparative particle size distribution data for milled and unmilled abiraterone acetate (volume statistics)**

| | D₁₀ (µm) | D₅₀ (µm) | D₉₀ (µm) | D4,3 (µm) |
|---|---|---|---|---|
| Milled | 0.0858 | 0.215 | 0.657 | 0.490 |
| Unmilled | 8.18 | 21.8 | 47.3 | 28.1 |

### Comparative example

### 5: Dissolution of milled and unmilled abiraterone acetate powder blends

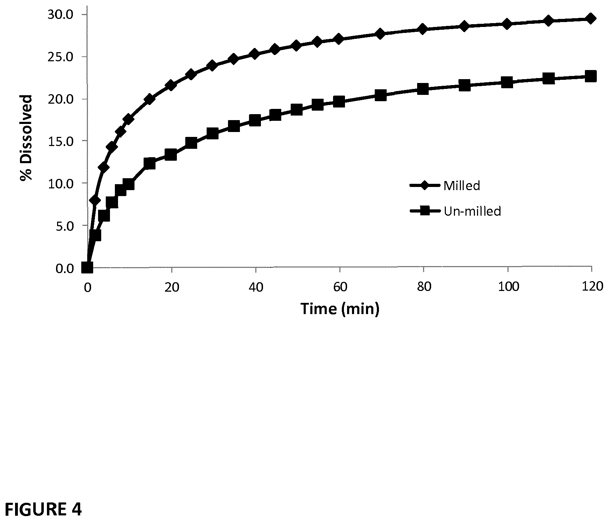

Dissolution behavior of the milled abiraterone acetate powder blend prepared in Example 3 was determined using an automated Sotax AT7 Smart dissolution testing unit fitted with a Thermo Fisher Scientific UV visible spectrometer (Model # EV0300 PC). The dissolution media was a 0.01 N HCl (pH=2) solution. The USP dissolution vessels were filled with 1000 ml of media and equilibrated to 37°C. The dissolution settings were according to USP type II apparatus with stirrer speed at 100 rpm. Two inline filters were utilized in series with pore sizes of 0.7 µm and 2.7 µm. The absorbance was measured at λ=236nm. Dissolution studies were performed by adding duplicate samples of milled and unmilled abiraterone acetate powder blends directly to the dissolution media. The unmilled powder blend was identical in composition to the milled powder blend but was not processed in the mill. An abiraterone acetate dose of 100 mg was used for milled and unmilled samples which corresponded to a total powder weight of 500.0 mg. For the powder dissolution studies, measurements were taken at 2, 4, 6, 8, 10, 15, 20, 25, 30, 35, 40, 45, 50, 55, 60, 70, 80, 90, 100, 110 and 120 minutes. The dissolution results are plotted as percent of abiraterone acetate dissolved. The results of this comparative study are presented in Table 2 and illustrated graphically in Figure 4. The results show that milled nanoparticulate abiraterone acetate dissolved more rapidly and to a greater percentage than the unmilled material.

**Table 2: Comparative dissolution rates of milled fine particle abiraterone acetate and unmilled abiraterone acetate (n=2)**

| | **Milled Abiraterone acetate (%)** | | **Unmilled Abiraterone acetate(%)** | |
|---|---|---|---|---|
| **Time (min)** | Average % Dissolved | Standard Deviation | Average % Dissolved | Standard Deviation |
| 0 | 0.0 | 0.0 | 0.0 | 0.0 |
| 2 | 8.0 | 0.0 | 3.8 | 0.3 |
| 4 | 11.9 | 0.7 | 6.2 | 0.5 |
| 6 | 14.3 | 1.0 | 7.7 | 0.6 |
| 8 | 16.1 | 0.9 | 9.2 | 0.6 |
| 10 | 17.6 | 1.1 | 9.9 | 0.0 |
| 15 | 19.9 | 0.7 | 12.3 | 1.0 |
| 20 | 21.5 | 0.5 | 13.3 | 0.6 |
| 25 | 22.8 | 0.3 | 14.7 | 0.9 |
| 30 | 23.8 | 0.2 | 15.8 | 0.8 |
| 35 | 24.6 | 0.1 | 16.7 | 0.7 |
| 40 | 25.2 | 0.2 | 17.3 | 0.4 |
| 45 | 25.7 | 0.3 | 18.0 | 0.3 |
| 50 | 26.2 | 0.4 | 18.6 | 0.2 |
| 55 | 26.6 | 0.6 | 19.2 | 0.1 |
| 60 | 26.9 | 0.7 | 19.5 | 0.1 |
| 70 | 27.6 | 0.8 | 20.3 | 0.2 |
| 80 | 28.0 | 0.9 | 21.0 | 0.2 |
| 90 | 28.4 | 1.0 | 21.4 | 0.3 |
| 100 | 28.7 | 1.0 | 21.8 | 0.3 |
| 110 | 29.0 | 1.1 | 22.2 | 0.3 |
| 120 | 29.3 | 1.2 | 22.5 | 0.3 |

### Example 6: Stability of Abiraterone Acetate Powder Blends

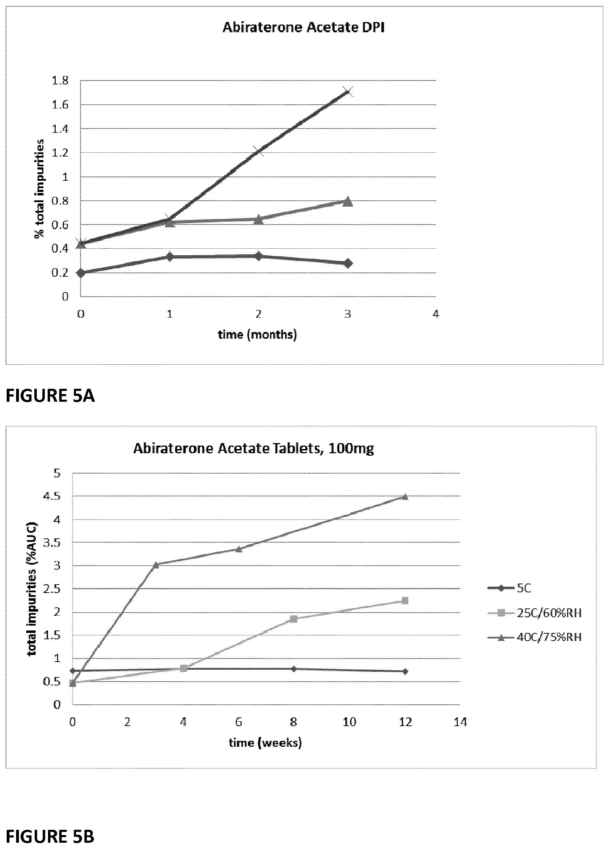

When abiraterone acetate particles larger than several microns in diameter was dry milled with lactose monohydrate and sodium lauryl sulfate then tested for impurities, **0**.4-0.6% total impurities was detected (%AUC). When this milled drug product intermediate (DPI) was further processed into tablets, the level of impurities was found to be higher, about 0.5-1.1%. Stability testing showed that the impurities grew at 25°C/60%RH and at 40°C/75%RH, but did not grow at 2-8°C. In addition, impurity growth in the tablets was faster than that in the milled DPI. Figure 5A (diamonds, 5°C; triangles, 25°C/60% RH; and crosses, 40∘C/75% RH) and Figure 5B (diamonds, SoC; squares, 25∘C/60% RH and triangles, 40∘C/75% RH) provide an overview of the impurity levels in lots of milled DPI and tablets, respectively upon accelerated stability testing. Tablets packaged with a nitrogen purge and stored refrigerated had an acceptably low level of impurities, but it is desirable to have formulation that can be started under ambient conditions.

The observed increase in impurities was compared to Zytiga^{®}, and the increase in impurities seen with the DPI and tablets comtaining fine powder abiraterone acetate was found to be greater than that observed with Zytiga^{®}.

The higher level of in impurities in tablets containing fine particle abiraterone acetate compared to Zytiga^{®} could arise from a number of sources, including, but not limited to: greater surface area of the API, higher proportion of excipients, and difference in excipients.

As part of the analysis of impurity growth, the impact on impurity level of various excipients useful for tablet preparation was examined by blending the DPI with various excipients and heating for 4 hours at 80°C. Microcrystalline cellulose (MCC), sodium lauryl sulfate (SLS), crosscarmellose sodium (CCS), sodium steryl fumarate (SSF), magnesium stetarate and hydrogentate vegetable oil were found to be associated with a lower level of impurity growth than pregelatiniaedstrach, spray dried lactose, poloxamer 188, crosspovidone, sodium starch glycolate. However, a mixture of MCC, SLS, CCS and SSF appeared to have a more detrimental impact on stability than might be suggested by the indivual impact of the components.

Additional studies indicated that the observed impurities appear to be degradation products of abiraterone acetate. Analysis if the API prior to size reduction showed essentially no degradation after 4 hours at 80°C, and minimal impurity growth when unmilled API is mixed with the excipients and heated. Further studies were conducted and it was concluded that degradation is not due to simply an interaction with the excipients or milling alone, but a combined effect.

### Example 5: Milling with Antioxidant or Sequestering Agent

Dry milling of abiraterone acetate was carried out in the presence of lactose monohydrate and sodium lauryl sulfate and various antioxidants and/or sequestering agents. Thus, in one study the dry milling also included a combination of ascorbic acid and fumaric acid or a combination of butylated Hydroxyanisole (BHA) and butylated Hydroxytoluene (BHT). This study produced DPI Formula Ascorbic/Fumaric and DPI Formula BHA/BHT, as shown in Table 3. Both DPI Formula contained abiraterone acetate having a [D90] below 1,000 nm and thus contain fine particle abiraterone acetate

**Table 3: DPI Formula Containing Antioxdant or Sequestering Agent**

| | | **DPI Formula Ascorbic/Fumaric** | **DPI Formula BHA/BHT** |
|---|---|---|---|
| **Ingredient** | **Function** | **% w/w** | **% w/w** |
| Abiraterone Acetate | Active | 30.00 | 30.00 |
| Lactose Monohydrate | Grinding compound | 67.35 | 67.65 |
| Sodium lauryl sulfate | Facilitating agent | 2.25 | 2.25 |
| Ascorbic acid | Antioxidant | 0.20 | |
| Fumaric acid | Sequestering agent | 0.20 | |
| Butylated Hydroxyanisole (BHA) | Antioxidant | | 0.05 |
| Butylated Hydroxytoluene (BHT) | Antioxidant | | 0.05 |
| total | | 100.00 | 100.00 |

The stability of the two DPI Formula in Table 3 were tested under accelerated conditions (4 hrs at 80°C). For DPI Formula Ascorbic/Fumaric, total impurities grew from 0.23 to only 0.80. For DPI Formula BHA/BHT, total impurities did not grow (0.38 both before and after 4 hrs at 80°C). In contrast, a DPI Formula in which abiraterone acetate wasdry milled with only lactose monohydrate and sodium lauryl sulfate (no antioxidant or sequestering agent), impurities grew from 1.63 initially to 3.86 after 4 hrs at 80°C.

The two different DPI Formulas were used to prepare two different corresponding tablet Formula as detailed in Table 4 by adding the indicated excipients to the DPI Formula, dry granulating and tableting.

**Table 4: DPI Tablet Containing Antioxdant or Sequestering Agent**

| | | Tablet Formula Ascorbic/Fumaric | Tablet Formula BHA/BHT |
|---|---|---|---|
| | Function | % w/w | % w/w |
| DPI **Formula Ascorbic/Fumaric** | | 47.62 | |
| (abirateroe acetate, lactose monohydrate, SLS, **ascorbic acid, fumaric acid**) | | | |
| DPI **Formula BHA/BHT** | | | 47.62 |
| (abiraterone acetate, lactose monohydrate, SLS, **BHA**, **BHT**) | | | |
| Microcrystalline cellulose | Diluent, disintegrant, compaction aid | 44.53 | 44.53 |
| Sodium lauryl sulfate | Wetting agent | 0.35 | 0.35 |
| Croscarmellose sodium | Disintegrant | 7.0 | 7.0 |
| Sodium stearyl fumarate | Lubricant | 0.5 | 0.5 |
| Total | | 100 | 100 |

The stability of the two Tablets Formula was tested under accelerated conditions (4 hrs at 80°C). In for Tablet Formula Ascorbic/Fumaric total impurities grew from 0.31 to only 0.38. For Tablet Formula BHA/BHT total impurities grew from 0.41 to only 0.44. This demonstrates that the addition of antioxidants and/or sequestering agent during milling can dramatically improve stability.

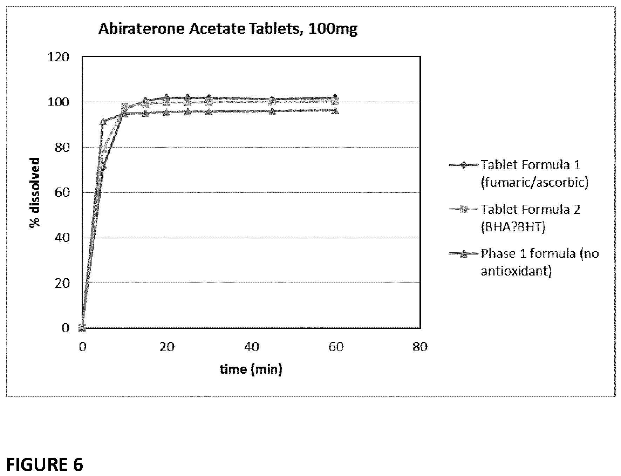

The dissolution rate of the abiraterone acetate in the Tablet Ascorbic/Fumaric and Tablet Formula BHA/BHT was tested using USP Apptartus II at 75 rpm (900 ml of pH 4.5 phosphate buffer (0.1% SLS)). Also tested was a similar formulation in which neither BHT/BHA or fumaric acid/ascorbic acid was present during milling (Tablet Formula 3). As shown in Figure 6 (triangles, Tablet Formula 3; squares, Tablet Formula BHA/BHT; diamonds, Tablet Formula ascorbic/fumaric), for all three types of tablets, 80% - 90% of the abiraterone acetate dissolved within 10 minutes.

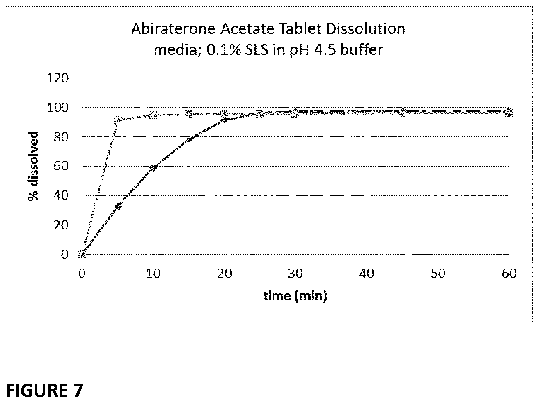

In order to compare the dissolution rate to a conventional abiraterone acetate formulation, a 250mg Zytiga tablet, cut down to an equivalent 100 mg weight was tested under the dissolution conditions described above alongside Tablet Formula without antioxidants or sequestering agent. As can be seen in Figure 7 (diamonds, 100 mg Zytiga tablet fragment; squares Tablet Formula 3), the Tablet Formula without antioxidants or sequestering agent, containing fine particle abiraterone acetate dissolved far more rapidly than the 100mg portion of the Zytiga tablet.

## Claims

1. A unit dosage pharmaceutical composition comprising:
(i) abiraterone acetate;
(ii) one or more of a millable grinding compound selected from lactose monohydrate, lactose anhydrous, and mannitol;
(iii) one or more of a facilitating agent selected from sodium lauryl sulfate, povidone and a stearic acid or salts or esters thereof; and
(iv) one or more of an antioxidant selected from ascorbic acid, butylated hydroxyanisole (BHA), and butylated hydroxytoluene (BHT) and/or one or more of a sequestering agent selected from fumaric acid, tartaric acid, and citric acid;
wherein [D₅₀] determined on a particle volume basis of the abiraterone acetate in the composition is greater than 100nm and less than one of: 1000nm, 900nm, 800nm, 700nm, 600nm, 500nm, 400 nm, 300nm, and 200nm;
wherein the composition is produced by dry milling; and
wherein the dissolution rate of the abiraterone acetate in the composition is such that when a sample containing 100mg of abiraterone acetate is tested in 900 ml of pH 4.5 phosphate buffer comprising 0.1% sodium lauryl sulfate (SLS), using USP Apparatus II at 75 rpm, at least 80% of the abiraterone acetate dissolves in 15 min or less, or in 10 min or less.

2. The unit dosage pharmaceutical composition of claim 1, wherein [D₉₀] determined on a particle volume basis of the abiraterone acetate is greater than 100nm and is less than one of: 1000nm, 900nm, 800nm, 700nm, 600nm, 500nm, 400nm, 300nm, and 200nm.

3. The unit dosage pharmaceutical composition of any of claims 1 to 2, wherein the unit dosage pharmaceutical composition is a tablet containing 10-400mg of abiraterone acetate

4. The unit dosage pharmaceutical composition of any of claims 1 to 3, wherein the mean AUC_{0-∞} of the unit dosage composition when administered to an adult male with a low-fat meal (7% fat, 300 calories) is 2-fold or less higher than when administered in the fasted state.

5. The unit dosage pharmaceutical composition of any of claims 1 to 4, wherein the mean AUC_{0-∞} of the unit dosage composition when administered to an adult male with a high-fat meal (57% fat, 825 calories) is 5-fold or less higher than when administered in the fasted state.

6. The unit dosage pharmaceutical composition of any of claims 1 to 5, wherein the mean Cmax of the unit dosage composition when administered to an adult male with a low-fat meal (7% fat, 300 calories) is 2-fold or less higher than when administered in the fasted state.

7. The unit dosage pharmaceutical composition of any of claims 1 to 6, wherein the mean Cmax of the unit dosage composition when administered to an adult male with a high-fat meal (57% fat, 825 calories) is 5-fold or less higher than when administered in the fasted state.

8. The unit dosage pharmaceutical composition of claim 1 for use in treating castration resistant prostate cancer, wherein a daily dose of 100-900 mg of abiraterone acetate is administered.

9. The unit dosage pharmaceutical composition for use of claim 8, wherein a daily dose of 200-500 mg of abiraterone acetate is administered.

10. The unit dosage pharmaceutical composition for use of claim 9, wherein a daily dose of 300-500 mg of abiraterone acetate is administered.

11. The unit dosage pharmaceutical composition for use of any one of claims 8 to 10, further comprising administering a glucocorticoid selected from prednisone, prednisolone, and methylprednisolone.

12. The unit dosage pharmaceutical composition for use of claim 10, wherein the glucocorticoid is methylprednisolone.

13. The unit dosage pharmaceutical composition of any of claims 1 to 7 or the unit dosage pharmaceutical composition for use of claims 8 to 12, wherein the facilitating agent is sodium lauryl sulfate or povidone.

14. The unit dosage pharmaceutical composition of any of claims 1 to 7 or the unit dosage pharmaceutical composition for use of claims 8 to 12, wherein the millable grinding compound is lactose monohydrate, the facilitating agent is sodium lauryl sulfate, and the antioxidant is selected from one or more of butylated hydroxyanisole, butylated hydroxytoluene and ascorbic acid.

## Patentansprüche

1. Pharmazeutische Einheitsdosiszusammensetzung, umfassend:
(i) Abirateronacetat;
(ii) eine oder mehrere mahlbare Mahlverbindungen, ausgewählt aus Lactosemonohydrat, wasserfreier Lactose und Mannit;
(iii) ein oder mehrere Hilfsmittel, ausgewählt aus Natriumlaurylsulfat, Povidon und einer Stearinsäure oder Salzen oder Estern davon; und
(iv) ein oder mehrere Antioxidantien, ausgewählt aus Ascorbinsäure, butyliertem Hydroxyanisol (BHA) und butyliertem Hydroxytoluol (BHT) und/oder ein oder mehrere Komplexbildner, ausgewählt aus Fumarsäure, Weinsäure und Zitronensäure;
wobei [D₅₀], bestimmt auf einer Partikelvolumenbasis des Abirateronacetats in der Zusammensetzung, größer als 100 nm und kleiner als eines von 1000 nm, 900 nm, 800 nm, 700 nm, 600 nm, 500 nm, 400 nm, 300 nm und 200 nm ist;
wobei die Zusammensetzung durch Trockenmahlen hergestellt wird; und
wobei die Auflösungsgeschwindigkeit des Abirateronacetats in der Zusammensetzung derart ist, dass, wenn eine Probe, die 100 mg Abirateronacetat enthält, in 900 ml Phosphatpuffer mit pH 4,5, der 0,1 % Natriumlaurylsulfat (SLS) umfasst, unter Verwendung der USP-Typ-II-Apparatur bei 75 U/min getestet wird, mindestens 80 % des Abirateronacetats sich in 15 min oder weniger oder 10 min oder weniger auflöst.

2. Pharmazeutische Einheitsdosiszusammensetzung nach
Anspruch 1, wobei [D₉₀], bestimmt auf einer Partikelvolumenbasis des Abirateronacetats, größer als 100 nm und kleiner als eines von 1000 nm, 900 nm, 800 nm, 700 nm, 600 nm, 500 nm, 400 nm, 300 nm und 200 nm ist.

3. Pharmazeutische Einheitsdosiszusammensetzung nach einem der Ansprüche 1 bis 2, wobei die pharmazeutische Einheitsdosiszusammensetzung eine Tablette ist, die 10-400 mg Abirateronacetat enthält.

4. Pharmazeutische Einheitsdosiszusammensetzung nach einem der Ansprüche 1 bis 3, wobei das mittlere AUC_{0-∞} der Einheitsdosiszusammensetzung, wenn sie einem männlichen Erwachsenen zusammen mit einer fettarmen Mahlzeit (7 % Fett, 300 Kalorien) verabreicht wird, um das 2-fache oder weniger höher ist, als wenn sie im nüchternen Zustand verabreicht wird.

5. Pharmazeutische Einheitsdosiszusammensetzung nach einem der Ansprüche 1 bis 4, wobei das mittlere AUC_{0-∞} der Einheitsdosiszusammensetzung, wenn sie einem männlichen Erwachsenen zusammen mit einer fettreichen Mahlzeit (57 % Fett, 825 Kalorien) verabreicht wird, um das 5-fache oder weniger höher ist, als wenn sie im nüchternen Zustand verabreicht wird.

6. Pharmazeutische Einheitsdosiszusammensetzung nach einem der Ansprüche 1 bis 5, wobei das mittlere Cmax der Einheitsdosiszusammensetzung, wenn sie einem männlichen Erwachsenen zusammen mit einer fettarmen Mahlzeit (7 % Fett, 300 Kalorien) verabreicht wird, um das 2-fache oder weniger höher ist, als wenn sie im nüchternen Zustand verabreicht wird.

7. Pharmazeutische Einheitsdosiszusammensetzung nach einem der Ansprüche 1 bis 6, wobei das mittlere Cmax der Einheitsdosiszusammensetzung, wenn sie einem männlichen Erwachsenen zusammen mit einer fettreichen Mahlzeit (57 % Fett, 825 Kalorien) verabreicht wird, um das 5-fache oder weniger höher ist, als wenn sie im nüchternen Zustand verabreicht wird.

8. Pharmazeutische Einheitsdosiszusammensetzung nach Anspruch 1 zur Verwendung bei der Behandlung von kastrationsresistentem Prostatakrebs, wobei eine tägliche Dosis von 100-900 mg Abirateronacetat verabreicht wird.

9. Pharmazeutische Einheitsdosiszusammensetzung zur Verwendung nach Anspruch 8, wobei eine tägliche Dosis von 200-500 mg Abirateronacetat verabreicht wird.

10. Pharmazeutische Einheitsdosiszusammensetzung zur Verwendung nach Anspruch 9, wobei eine tägliche Dosis von 300-500 mg Abirateronacetat verabreicht wird.

11. Pharmazeutische Einheitsdosiszusammensetzung zur Verwendung nach einem der Ansprüche 8 bis 10, ferner umfassend die Verabreichung eines Glucocorticoids, ausgewählt aus Prednison, Prednisolon und Methylprednisolon.

12. Pharmazeutische Einheitsdosiszusammensetzung zur Verwendung nach Anspruch 10, wobei das Glucocorticoid Methylprednisolon ist.

13. Pharmazeutische Einheitsdosiszusammensetzung nach einem der Ansprüche 1 bis 7 oder pharmazeutische Einheitsdosiszusammensetzung zur Verwendung nach den Ansprüchen 8 bis 12, wobei das Hilfsmittel Natriumlaurylsulfat oder Povidon ist.

14. Pharmazeutische Einheitsdosiszusammensetzung nach einem der Ansprüche 1 bis 7 oder pharmazeutische Einheitsdosiszusammensetzung zur Verwendung nach den Ansprüchen 8 bis 12, wobei die mahlbare Mahlverbindung Lactosemonohydrat ist, das Hilfsmittel Natriumlaurylsulfat ist und das Antioxidans ausgewählt ist aus einem oder mehreren von butyliertem Hydroxyanisol, butyliertem Hydroxytoluol und Ascorbinsäure.

## Revendications

1. Composition pharmaceutique en dose unitaire comprenant :
(i) de l'acétate d'abiratérone ;
(ii) un ou plusieurs composés de trituration broyables choisis parmi le lactose monohydraté, le lactose anhydre et le mannitol ;
(iii) une ou plusieurs substances auxiliaires choisies parmi le laurylsulfate de sodium, la povidone et un acide stéarique ou des sels ou esters de ceux-ci ; et
(iv) un ou plusieurs antioxydants choisis parmi l'acide ascorbique, l'hydroxyanisole butylé (BHA), et l'hydroxytoluène butylé (BHT) et/ou un ou plusieurs agents séquestrants choisis parmi l'acide fumarique, l'acide tartrique, et l'acide citrique ;
dans laquelle [D₅₀] déterminé sur une base de volume de particules de l'acétate d'abiratérone dans la composition est supérieur à 100 nm et inférieur à l'un parmi : 1000 nm, 900 nm, 800 nm, 700 nm, 600 nm, 500 nm, 400 nm, 300 nm, et 200 nm ;
dans laquelle la composition est produite par broyage à sec ; et
dans laquelle la vitesse de dissolution de l'acétate d'abiratérone dans la composition est telle que lorsqu'un échantillon contenant 100 mg d'acétate d'abiratérone est mis à l'essai dans 900 ml de tampon phosphate de pH 4,5 comprenant du laurylsulfate de sodium (SLS) à 0,1 %, à l'aide d'un appareil II USP à 75 tr/min, au moins 80 % de l'acétate d'abiratérone se dissout en 15 min ou moins, ou en 10 min ou moins.

2. Composition pharmaceutique en dose unitaire selon la revendication 1, dans laquelle [D₉₀] déterminé sur une base de volume de particules de l'acétate d'abiratérone est supérieur à 100 nm et est inférieur à l'un parmi : 1000 nm, 900 nm, 800 nm, 700 nm, 600 nm, 500 nm, 400 nm, 300 nm, et 200 nm.

3. Composition pharmaceutique en dose unitaire selon l'une quelconque des revendications 1 et 2, dans laquelle la composition pharmaceutique en dose unitaire est un comprimé contenant 10 à 400 mg d'acétate d'abiratérone.

4. Composition pharmaceutique en dose unitaire selon l'une quelconque des revendications 1 à 3, dans laquelle la moyenne AUC_{0-∞} de la composition en dose unitaire lorsqu'elle est administrée à un homme adulte avec un repas à faible teneur en matières grasses (7 % de matières grasses, 300 calories) est 2 fois supérieure ou moins à celle d'une administration à jeun.

5. Composition pharmaceutique en dose unitaire selon l'une quelconque des revendications 1 à 4, dans laquelle la moyenne AUC_{0-∞} de la composition en dose unitaire lorsqu'elle est administrée à un homme adulte avec un repas à haute teneur en matières grasses (57 % de matières grasses, 825 calories) est 5 fois supérieure ou moins à celle d'une administration à jeun.

6. Composition pharmaceutique en dose unitaire selon l'une quelconque des revendications 1 à 5, dans laquelle la moyenne Cmax de la composition en dose unitaire lorsqu'elle est administrée à un homme adulte avec un repas à faible teneur en matières grasses (7 % de matières grasses, 300 calories) est 2 fois supérieure ou moins à celle d'une administration à jeun.

7. Composition pharmaceutique en dose unitaire selon l'une quelconque des revendications 1 à 6, dans laquelle la moyenne Cmax de la composition en dose unitaire lorsqu'elle est administrée à un homme adulte avec un repas à haute teneur en matières grasses (57 % de matières grasses, 825 calories) est 5 fois supérieure ou moins à celle d'une administration à jeun.

8. Composition pharmaceutique en dose unitaire selon la revendication 1 pour une utilisation dans le traitement du cancer de la prostate résistant à la castration, dans laquelle une dose quotidienne de 100 à 900 mg d'acétate d'abiratérone est administrée.

9. Composition pharmaceutique en dose unitaire pour une utilisation selon la revendication 8, dans laquelle une dose quotidienne de 200 à 500 mg d'acétate d'abiratérone est administrée.

10. Composition pharmaceutique en dose unitaire pour une utilisation selon la revendication 9, dans laquelle une dose quotidienne de 300 à 500 mg d'acétate d'abiratérone est administrée.

11. Composition pharmaceutique en dose unitaire pour une utilisation selon l'une quelconque des revendications 8 à 10, comprenant en outre l'administration d'un glucocorticoïde choisi parmi la prednisone, la prednisolone, et la méthylprednisolone.

12. Composition pharmaceutique en dose unitaire pour une utilisation selon la revendication 10, dans laquelle le glucocorticoïde est la méthylprednisolone.

13. Composition pharmaceutique en dose unitaire selon l'une quelconque des revendications 1 à 7 ou composition pharmaceutique en dose unitaire pour une utilisation selon les revendications 8 à 12, dans laquelle la substance auxiliaire est le laurylsulfate de sodium ou la povidone.

14. Composition pharmaceutique en dose unitaire selon l'une quelconque des revendications 1 à 7 ou composition pharmaceutique en dose unitaire pour une utilisation selon les revendications 8 à 12, dans laquelle le composé de trituration broyable est le lactose monohydraté, la substance auxiliaire est le laurylsulfate de sodium, et l'antioxydant est choisi parmi un ou plusieurs parmi l'hydroxyanisole butylé, l'hydroxytoluène butylé et l'acide ascorbique.
